# EUROPEAN PATENT SPECIFICATION

(11) **EP 1 408 736 B1**
(45) Date of publication and mention of the grant of the patent: **21.10.2009**
(21) Application number: 02720425.4
(22) Date of filing: 26.03.2002
(51) Int. Cl.: A01H 5/00, C12N 15/82, C12N 15/84, C12N 15/87

(54) **PLANTS CHARACTERIZED BY ENHANCED GROWTH AND METHODS AND NUCLEIC ACID CONSTRUCTS USEFUL FOR GENERATING SAME**
DURCH VERSTÄRKTES WACHSTUM CHARAKTERISIERTE PFLANZEN SOWIE FÜR DEREN ERZEUGUNG GEEIGNETE VERFAHREN UND NUKLEINSÄUREKONSTRUKTE
PLANTES CARACTERISEES PAR UNE CROISSANCE AMELIOREE, PROCEDES ET CONSTRUCTIONS D'ACIDE NUCLEIQUE UTILES DANS LA PRODUCTION DE CES PLANTES

(30) Priority: 09.04.2001 US 828173
(43) Date of publication of application: 21.04.2004
(73) Proprietor: Yissum Research Development Company, of The Hebrew University of Jerusalem, 91390 Jerusalem (IL)
(72) Inventor: KAPLAN, Aaron, 97277 Jerusalem (IL); LIEMAN-HURWITZ, Judy, 97791 Jerusalem (IL); SCHATZ, Daniella, 93320 Jerusalem (IL); MITTLER, Ron, 97440 Jerusalem (IL); RACHMILEVITCH, Shimon, 52653 Ramat Gan (IL)
(74) Representative: Vossius & Partner
(86) International application number: PCT/IL2002/000250
(87) International publication number: WO 2002/081622

(56) References cited:
- US-B1- 6 320 101
- KAPLAN AARON ET AL: "CO2 concentrating mechanisms in photosynthetic microorganisms" ANNUAL REVIEW OF PLANT PHYSIOLOGY AND PLANT MOLECULAR BIOLOGY ANNUAL REVIEWS INC. {A}, P.O. BOX 10139, 4139 EL CAMINO WAY, PALO ALTO, CALIFORNIA 94306, USA SERIES : ANNUAL REVIEW OF PLANT PHYSIOLOGY AND PLANT MOLECULAR BIOLOGY (ISSN 1040-2519), 1999, pages 539-570, XP002303190 ISSN: 0-8243-0650-3
- GHOSHAL DURBA ET AL: "Carbon concentration mechanism(s) in unicellular green algae and cyanobacteria" JOURNAL OF PLANT BIOCHEMISTRY AND BIOTECHNOLOGY, vol. 10, no. 2, July 2001 (2001-07), pages 83-90, XP009039056 ISSN: 0971-7811
- MAEDA S. ET AL: 'Bicarbonate binding activity of the CmpA protein of the cyanobacterium synechococcus sp. strain PCC 7942 involved in active transport of bicarbonate' THE JOURNAL OF BIOLOGICAL CHEMISTRY vol. 275, no. 27, 07 June 2000, pages 20551 - 20555, XP002979839
- DATABASE GENBANK [Online] 18 September 1996 BONFIL ET AL, XP002979840 Retrieved from NCBI Database accession no. (U62616)
- OMATA T. ET AL: 'Identification of an ATP-binding cassette transporter involved in bicarbonate uptake in the cyanobacterium synechococcus sp. strain PCC 7942' PROC. NATL. ACAD. SCI. USA vol. 96, no. 23, 09 November 1999, pages 13571 - 13576, XP002979841
- SCHLEGEL H.G.: 'Allgemeine Mikrobiologie', 1981, GEORG THIEME VERLAG pages 108 - 109 * pages 108-109 *
- ZHANG C.-C. ET AL: 'Survey, analysis and genetic organization of genes encoding eukaryotik-like signaling proteins on a cyanobacterial genome' NUCLEIC ACIDS RESEARCH vol. 26, no. 16, 1998, pages 3619 - 3625
- WHITTAKER R.H.; MARGULIS L.: "Protist classification and the kingdomsof organisms" BIOSYSTEMS, vol. 10, 1978, pages 3-18,

### SEQUENCE LISTING

<110> Kaplan, Aaron
   Lieman-Hurwitz, Judy
   Rachmilevitch, Shimon
   Schatz, Daniella
   Mittler, Ron
<120> PLANTS CHARACTERIZED BY ENHANCED GROWTH AND METHODS AND NUCLEIC ACID CONSTRUCTS USEFUL FOR GENERATING SAME
<130> 00/21536
<160> 9
<170> PatentIn version 3.0
<210> 1
   <211> 4957
   <212> DNA
   <213> Synechococcus sp.
<400> 1
   aagcttggat tgaagcgatc ggggtcaatc ccagcgatga tcctcagttc ctcctgatgg     60
   tcgatccctt tagcgccaag attgaggatc tgctgcaagg gctggatttc gcctatcccg    120
   aggccgtgaa agtgggcgga ttggccagtg gtttgggggc agagtcagcg atcgccagct    180
   tgttttttca agaccgacag gtcgatggcg tgattgggct agccctcagt ggcaatgtcc    240
   agctgcaggc gatcgtggct cagggctgtc gtccagttgg cccgctttgg catgtggcag    300
   cggcggagcg caacattctg cggcaacttc agaccgaaga cgaggaaccg atcgccgcgc    360
   tgcaagccct acagtcagtc ctgcgtgatc tctcccctga attacagcga tcgctctgtg    420
   tgggcctggc ctgcaattct ttccaaacgg tattacaacc gggcgacttc ctgatccgta    480
   acctgctggg gtttgatccc cgcactggtg ctgtagcaat cggcgatcgc attcgagttg    540
   ggcagcggct gcagctgcac gtacgggatg cccagacagc ggcggatgac ctcgagcggc    600
   aactggggca atggtgccgg cagcatgcga caaaaccagc agcttccctc ttgttttcct    660
   gcttggggcg cggcaagccc ttctatcagc aggccaactt cgagtcgcaa ctgattcagc    720
   attacctctc agagctgccc ctagctggct ttttctgtaa tggcgaaatc ggcccgatcg    780
   ctggcagcac ctacctgcat ggctacacat cggtgctggc tttgctgtcg gccaaaactc    840
   actagcgcca gcgagacctg attgtcgatc tgctgagcgc gactgtagcg ctggaaatag    900
   gcccggacct gagcaggcgc atcggccaag ctgaccgtag tatcaccgtc agccaccccc    960
   gcccagaaat tccgcaacat cggcaggaga gcgatcgcct ccgcctccga taaattcaac   1020
   ggctcatggg tcaacaggcg gatcaagtac tctgactgcg atcgccatcc attcccgccg   1080
   aaaacgtttg taaatcagtc ttgatccggt agcgatcgca cccgacggga ctctagttct 1140
   agttgccaac cttcagcggc aggttgtacg gttccgagtc ggtagggatg gggatagctg 1200
   accaaggaac cggtcgtgac ttcccagaga gcaccttgct gactggtggc ttggatgtgg 1260
   aggtggcctg tgaagatcac cgagacgctg cccgcttcga ggattgatcg caattcctcg 1320
   gcattttcta agatgtagcg ctgaccaagc ggatgctgct gttgatcggg cagatgctcc 1380
   aacacattgt ggtgaatcat cacccagcgt tggctagcgg tggaagtggc gagttcttgt 1440
   tgcagccagt tgagttgcgc gcaatcgact cgcccccgat gcagttgatg gcccgcttca 1500
   tcaaaagcga tcgaattcag cgcaaacaga tcgagatccg gtgcgatcgt gcagcgatag 1560
   taggggcgat cgctcgtgaa gccaaagtct tgatagagct cgacaaactc ggccacaccg 1620
   gtgcgatcgc gatcgctcgc tgcggcgggc atatcgtggt tgcccggcac cacatagacc 1680
   ggatagggca actggcgcaa ttgttgcagc agccactgat ggttttcccg ctccccgtgc 1740
   tgggttaaat cccccggcag caacaggaag tccaaatcca gcgctgccag ttctgtcagg 1800
   atttgctcaa aagccggaat gctgcactca atcaaatgga agcgatgggg atggtgccaa 1860
   attgtctgcg gcagtccaat gtggagatcg ctcagcagcg caaatcgaaa cgctcggttc 1920
   attgccatcc cctcagctat cgagcccgat tctaggcgaa gctaggtcga gtccgttgtc 1980
   ttcagttgca agcattcatg gccagagttc gcgttcggca gcacgtcaat ccgctctctc 2040
   agaaattcca agtggtcacg acttggccgg attggcaaca ggtctatgcg gactgcgatc 2100
   gcccgctgca tttggatatt ggctgtgctc gcgggcgctt tctgctggca atggcgacac 2160
   gacaacctga gtggaattat ctggggctgg aaattcgtga gccgctggta gatgaggcga 2220
   acgcgatcgc ccgcgaacgt gaactgacca atctctacta ccacttcagc aacgccaatt 2280
   tggacttgga accgctgctg cgatcgctgc cgacagggat tttgcagcgg gtcagcattc 2340
   agttcccgga tccttggttc aagaaacgcc atcaaaagcg acgcgtcgtc cagccggaac 2400
   tggtgcaagc cctcgcgact gcgttacctg ctggtgcaga ggtctttctg caatccgatg 2460
   tgctggaagt gcaggcagag atgtgcgaac actttgcggc ggaaccccgc tttcagcgca 2520
   cctgcttgga ctggctgccg gaaaatccgc tgcccgtccc gaccgagcgc gaaattgccg 2580
   ttcaaaacaa acagttgcca gtctaccgtg ctctcttcat tcggcagcca gcggactaag 2640
   ctcttaaggc aagcgttgac gcgatcgcga tgactgtctg gcaaactctg acttttgccc 2700
   attaccaacc ccaacagtgg ggccacagca gtttcttgca tcggctgttt ggcagcctgc 2760
   gagcttggcg ggcctccagc cagctgttgg tttggtctga ggcactgggt ggcttcttgc 2820
   ttgctgtcgt ctacggttcg gctccgtttg tgcccagttc cgccctaggg ttggggctag 2880
   ccgcgatcgc ggcctattgg gccctgctct cgctgacaga tatcgatctg cggcaagcaa 2940
   cccccattca ctggctggtg ctgctctact ggggcgtcga tgccctagca acgggactct 3000
   cacccgtacg cgctgcagct ttagttgggc tagccaaact gacgctctac ctgttggttt 3060
   ttgccctagc ggctcgggtt ctccgcaatc cccgtctgcg atcgctgctg ttctcggtcg 3120
   tcgtgatcac atcgcttttt gtcagtgtct acggcctcaa ccaatggatc tacggcgttg 3180
   aagagctggc gacttgggtg gatcgcaact cggttgccga cttcacctca cgggtttaca 3240
   gctatctggg caaccccaac ctgctggctg cttatctggt gccgacgact gccttttctg 3300
   cagcagcgat cggggtgtgg cgcggctggc tccccaagct gctggcgatc gctgcgacag 3360
   gtgcgagcag cttatgtctg atcctcacct acagtcgcgg tggctggctg ggttttgtcg 3420
   ccatgatttt tgtctgggcg ttattagggc tctactggtt tcaaccccgt ctacccgcac 3480
   cctggcgacg ctggctattc ccagtcgtat tgggtggact agtcgcggtg ctcttggtgg    3540
   cggtgcttgg acttgagccg ttgcgcgtgc gcgtgttgag catctttgtg gggcgtgaag    3600
   acagcagcaa caacttccgg atcaatgtct ggctggcggt gctgcagatg attcaagatc    3660
   ggccttggct gggcatcggc cccggcaata ccgcctttaa cctggtttat cccctctatc    3720
   aacaggcgcg ctttacggcg ttgagcgcct actccgtccc gctggaagtc gcggttgagg    3780
   gcggactact gggcttgacg gccttcgctt ggctgctgct ggtcacggcg gtgacggcgg    3840
   tgcggcaggt gagccgactg cggcgcgatc gcaatcccca agccttttgg ttgatggcta    3900
   gcttggccgg tttggcagga atgctgggtc acggtctgtt tgataccgtg ctctatcgac    3960
   cggaagccag tacgctctgg tggctctgta ttggagcgat cgcgagtttc tggcagcccc    4020
   aaccttccaa gcaactccct ccagaagccg agcattcaga cgaaaaaatg tagcgggctc    4080
   cccaacaaat tcctgtgcac ccgactggat ccaccaccta aactggatcc caaaggtatc    4140
   cggtggatct agggtcataa cgaactccga ccgcgatcgc gtccgcgaac tgaacctcca    4200
   tcgcaccgaa gcggagttcg ttagtcgttg aagagccaat gctagagggg gctgccgaag    4260
   cagttgggct ggaagcaggc tgcgagaagc cacccgcatc caaggcaaag ttcagccgac    4320
   cttccgcaaa gactacgatc gccacggcgg ctctgccagc taagtcagcg ctgggttagt    4380
   tgtcatagca gtccgcagac aagttaggac aacttcatag agggactcgc tcagagtcaa    4440
   cagccgctgt ccgtgggggt gcgcaatcac ccccacaccc acgcactggg ggactcgact    4500
   cccccaggcc ccccgcaaca agatttcgga taaggggcat cggctgaatc gcgatcgctg    4560
   cgggtaaaac tagccggtgt tagccatggg tttgagacta atcggcacgg ggcaaaacgt    4620
   cctgatttat ttgctcaatg tgataggtta catcgtcaaa aacaaggccc aagaggtagg    4680
   aaaaatcacg accgcccaag tccgagggct ttgctgttgg gagcgaccta gggcagacta    4740
   gacagagcat tgctgtgagc caaagcgcct tcaattgctg gcggctgtgg gtttttcgga    4800
   ggttgccaaa tgaaagacct tttcgtcaat gtcctccgct atccccgcta cttcatcacc    4860
   ttccagctgg gtatttttta gtcgatctac cagtgggtgc ggccgatggt tcgcaaccca    4920
   gtcgcggctt gggcgctgct aggctttgga gtttcga                              4957
<210> 2
   <211> 1404
   <212> DNA
   <213> Synechococcus sp.
<400> 2
   atgactgtct ggcaaactct gacttttgcc cattaccaac cccaacagtg gggccacagc      60
   agtttcttgc atcggctgtt tggcagcctg cgagcttggc gggcctccag ccagctgttg     120
   gtttggtctg aggcactggg tggcttcttg cttgctgtcg tctacggttc ggctccgttt     180
   gtgcccagtt ccgccctagg gttggggcta gccgcgatcg cggcctattg ggccctgctc     240
   tcgctgacag atatcgatct gcggcaagca acccccattc actggctggt gctgctctac     300
   tggggcgtcg atgccctagc aacgggactc tcacccgtac gcgctgcagc tttagttggg     360
   ctagccaaac tgacgctcta cctgttggtt tttgccctag cggctcgggt tctccgcaat     420
   ccccgtctgc gatcgctgct gttctcggtc gtcgtgatca catcgctttt tgtcagtgtc     480
   tacggcctca accaatggat ctacggcgtt gaagagctgg cgacttgggt ggatcgcaac     540
   tcggttgccg acttcacctc acgggtttac agctatctgg gcaaccccaa cctgctggct 600
   gcttatctgg tgccgacgac tgccttttct gcagcagcga tcggggtgtg gcgcggctgg 660
   ctccccaagc tgctggcgat cgctgcgaca ggtgcgagca gcttatgtct gatcctcacc 720
   tacagtcgcg gtggctggct gggttttgtc gccatgattt ttgtctgggc gttattaggg 780
   ctctactggt ttcaaccccg tctacccgca ccctggcgac gctggctatt cccagtcgta 840
   ttgggtggac tagtcgcggt gctcttggtg gcggtgcttg gacttgagcc gttgcgcgtg 900
   cgcgtgttga gcatctttgt ggggcgtgaa gacagcagca acaacttccg gatcaatgtc 960
   tggctggcgg tgctgcagat gattcaagat cggccttggc tgggcatcgg ccccggcaat 1020
   accgccttta acctggttta tcccctctat caacaggcgc gctttacggc gttgagcgcc 1080
   tactccgtcc cgctggaagt cgcggttgag ggcggactac tgggcttgac ggccttcgct 1140
   tggctgctgc tggtcacggc ggtgacggcg gtgcggcagg tgagccgact gcggcgcgat 1200
   cgcaatcccc aagccttttg gttgatggct agcttggccg gtttggcagg aatgctgggt 1260
   cacggtctgt ttgataccgt gctctatcga ccggaagcca gtacgctctg gtggctctgt 1320
   attggagcga tcgcgagttt ctggcagccc caaccttcca agcaactccc tccagaagcc 1380
   gagcattcag acgaaaaaat gtag 1404
<210> 3
   <211> 467
   <212> PRT
   <213> Synechococcus sp.
<400> 3
   Met Thr Val Trp Gln Thr Leu Thr Phe Ala His Tyr Gln Pro Gln Gln
   1               5                   10                  15
   Trp Gly His Ser Ser Phe Leu His Arg Leu Phe Gly Ser Leu Arg Ala
               20                  25                  30
   Trp Arg Ala Ser Ser Gln Leu Leu Val Trp Ser Glu Ala Leu Gly Gly
           35                  40                  45
   Phe Leu Leu Ala Val Val Tyr Gly Ser Ala Pro Phe Val Pro Ser Ser
       50                  55                  60
   Ala Leu Gly Leu Gly Leu Ala Ala Ile Ala Ala Tyr Trp Ala Leu Leu
   65                  70                  75                  80
   Ser Leu Thr Asp Ile Asp Leu Arg Gln Ala Thr Pro Ile His Trp Leu
                   85                  90                  95
   Val Leu Leu Tyr Trp Gly Val Asp Ala Leu Ala Thr Gly Leu Ser Pro
               100                 105                 110
   Val Arg Ala Ala Ala Leu Val Gly Leu Ala Lys Leu Thr Leu Tyr Leu
           115                 120                 125
   Leu Val Phe Ala Leu Ala Ala Arg Val Leu Arg Asn Pro Arg Leu Arg
       130                 135                 140
   Ser Leu Leu Phe Ser Val Val Val Ile Thr Ser Leu Phe Val Ser Val
   145                 150                 155                 160
   Tyr Gly Leu Asn Gln Trp Ile Tyr Gly Val Glu Glu Leu Ala Thr Trp
                   165                 170                 175
   Val Asp Arg Asn Ser Val Ala Asp Phe Thr Ser Arg Val Tyr Ser Tyr
               180                 185                 190
   Leu Gly Asn Pro Asn Leu Leu Ala Ala Tyr Leu Val Pro Thr Thr Ala
   195 200 205
   Phe Ser Ala Ala Ala Ile Gly Val Trp Arg Gly Trp Leu Pro Lys Leu
   210 215 220
   Leu Ala Ile Ala Ala Thr Gly Ala Ser Ser Leu Cys Leu Ile Leu Thr
   225 230 235 240
   Tyr Ser Arg Gly Gly Trp Leu Gly Phe Val Ala Met Ile Phe Val Trp
   245 250 255
   Ala Leu Leu Gly Leu Tyr Trp Phe Gln Pro Arg Leu Pro Ala Pro Trp
   260 265 270
   Arg Arg Trp Leu Phe Pro Val Val Leu Gly Gly Leu Val Ala Val Leu
   275 280 285
   Leu Val Ala Val Leu Gly Leu Glu Pro Leu Arg Val Arg Val Leu Ser
   290 295 300
   Ile Phe Val Gly Arg Glu Asp Ser Ser Asn Asn Phe Arg Ile Asn Val
   305 310 315 320
   Trp Leu Ala Val Leu Gln Met Ile Gln Asp Arg Pro Trp Leu Gly Ile
   325 330 335
   Gly Pro Gly Asn Thr Ala Phe Asn Leu Val Tyr Pro Leu Tyr Gln Gln
   340 345 350
   Ala Arg Phe Thr Ala Leu Ser Ala Tyr Ser Val Pro Leu Glu Val Ala
   355 360 365
   Val Glu Gly Gly Leu Leu Gly Leu Thr Ala Phe Ala Trp Leu Leu Leu
   370 375 380
   Val Thr Ala Val Thr Ala Val Arg Gln Val Ser Arg Leu Arg Arg Asp
   385 390 395 400
   Arg Asn Pro Gln Ala Phe Trp Leu Met Ala Ser Leu Ala Gly Leu Ala
   405 410 415
   Gly Met Leu Gly His Gly Leu Phe Asp Thr Val Leu Tyr Arg Pro Glu
   420 425 430
   Ala Ser Thr Leu Trp Trp Leu Cys Ile Gly Ala Ile Ala Ser Phe Trp
   435 440 445
   Gln Pro Gln Pro Ser Lys Gln Leu Pro Pro Glu Ala Glu His Ser Asp
   450 455 460
   Glu Lys Met
   465
<210> 4
   <211> 1425
   <212> DNA
   <213> Synechocystis sp.
<400> 4
   atggtgtctc ccatctctat ctggcgatcg ctgatgtttg gcggtttttc cccccaggaa     60
   tggggccggg gcagtgtgct ccatcgtttg gtgggctggg gacagagttg gatacaggct    120
   agtgtgctct ggccccactt cgaggcattg ggtacggctc tagtggcaat aatttttatt    180
   gcggctccct tcacctccac caccatgttg ggcattttta tgctgctctg tggagccttt    240
   tgggctctgc tgacctttgc tgatcaacca gggaagggtt tgactcccat ccatgtttta    300
   gtttttgcct actggtgcat ttcggcgatc gccgtgggat tttctccggt aaaaatggcg    360
   gcggcgtcgg ggttagcgaa attaacagct aatttatgtc tgtttctact ggcggcgagg    420
   ttattgcaaa acaaacaatg gttgaaccgg ttagtaaccg ttgttttact ggtagggcta    480
   ttggtgggga gttacggtct gcgacaacag gtggacgggg tagaacagtt agccacttgg    540
   aatgacccca cctctacctt ggcccaggcc actagggtat atagcttttt aggtaatccc    600
   aatctcttgg cggcttacct ggtgcccatg acgggtttga gcttgagtgc cctggtggta    660
   tggcgacggt ggtggcccaa actgctggga gcaaccatgg tgattgttaa cctactctgt    720
   ctctttttta cccagagccg gggcggttgg ctagcagtgc tggccctggg agctaccttc    780
   ctggcccttt gttacttctg gtggttaccc caattaccca aattttggca acggtggtct    840
   ttgcccctgg cgatcgccgt ggcggttata ttaggtgggg gagcgttgat tgcggtggaa    900
   ccgattcgac tcagggccat gagcattttt gctgggcggg aagacagcag taataatttc    960
   cgcatcaatg tttgggaagg ggtaaaagcc atgatccgag cccgccctat cattggcatt   1020
   ggcccaggta acgaagcctt taaccaaatt tatccttact atatgcggcc ccgcttcacc   1080
   gccctgagtg cctattccat ttacctagaa attttggtgg aaacgggtgt agttggtttt   1140
   acctgtatgc tctggctgtt ggccgttacc ctaggcaaag gcgtagaact ggttaaacgc   1200
   tgtcgccaaa ccctcgcccc ggaaggcatc tggattatgg gggctttagc ggcgatcatc   1260
   ggtttgttgg tccacggcat ggtagataca gtctggtacc gtcccccggt gagcactttg   1320
   tggtggttgc tagtggccat tgttgctagt cagtgggcca gcgcccaggc ccgtttggag   1380
   gccagtaaag aagaaaatga ggacaaacct cttcttgctt cataa                   1425
<210> 5
   <211> 474
   <212> PRT
   <213> Synechocystis sp.
<400> 5
   Met Val Ser Pro Ile Ser Ile Trp Arg Ser Leu Met Phe Gly Gly Phe
   1               5                   10                  15
   Ser Pro Gln Glu Trp Gly Arg Gly Ser Val Leu His Arg Leu Val Gly
               20                  25                  30
   Trp Gly Gln Ser Trp Ile Gln Ala Ser Val Leu Trp Pro His Phe Glu
           35                  40                  45
   Ala Leu Gly Thr Ala Leu Val Ala Ile Ile Phe Ile Ala Ala Pro Phe
       50                  55                  60
   Thr Ser Thr Thr Met Leu Gly Ile Phe Met Leu Leu Cys Gly Ala Phe
   65                  70                  75                  80
   Trp Ala Leu Leu Thr Phe Ala Asp Gln Pro Gly Lys Gly Leu Thr Pro
                   85                  90                  95
   Ile His Val Leu Val Phe Ala Tyr Trp Cys Ile Ser Ala Ile Ala Val
               100                 105                 110
   Gly Phe Ser Pro Val Lys Met Ala Ala Ala Ser Gly Leu Ala Lys Leu
           115                 120                 125
   Thr Ala Asn Leu Cys Leu Phe Leu Leu Ala Ala Arg Leu Leu Gln Asn
       130                 135                 140
   Lys Gln Trp Leu Asn Arg Leu Val Thr Val Val Leu Leu Val Gly Leu
   145                 150                 155                 160
   Leu Val Gly Ser Tyr Gly Leu Arg Gln Gln Val Asp Gly Val Glu Gln
                   165                 170                 175
   Leu Ala Thr Trp Asn Asp Pro Thr Ser Thr Leu Ala Gln Ala Thr Arg
               180                 185                 190
   Val Tyr Ser Phe Leu Gly Asn Pro Asn Leu Leu Ala Ala Tyr Leu Val
   195 200 205
   Pro Met Thr Gly Leu Ser Leu Ser Ala Leu Val Val Trp Arg Arg Trp
   210 215 220
   Trp Pro Lys Leu Leu Gly Ala Thr Met Val Ile Val Asn Leu Leu Cys
   225 230 235 240
   Leu Phe Phe Thr Gln Ser Arg Gly Gly Trp Leu Ala Val Leu Ala Leu
   245 250 255
   Gly Ala Thr Phe Leu Ala Leu Cys Tyr Phe Trp Trp Leu Pro Gln Leu
   260 265 270
   Pro Lys Phe Trp Gln Arg Trp Ser Leu Pro Leu Ala Ile Ala Val Ala
   275 280 285
   Val Ile Leu Gly Gly Gly Ala Leu Ile Ala Val Glu Pro Ile Arg Leu
   290 295 300
   Arg Ala Met Ser Ile Phe Ala Gly Arg Glu Asp Ser Ser Asn Asn Phe
   305 310 315 320
   Arg Ile Asn Val Trp Glu Gly Val Lys Ala Met Ile Arg Ala Arg Pro
   325 330 335
   Ile Ile Gly Ile Gly Pro Gly Asn Glu Ala Phe Asn Gln Ile Tyr Pro
   340 345 350
   Tyr Tyr Met Arg Pro Arg Phe Thr Ala Leu Ser Ala Tyr Ser Ile Tyr
   355 360 365
   Leu Glu Ile Leu Val Glu Thr Gly Val Val Gly Phe Thr Cys Met Leu
   370 375 380
   Trp Leu Leu Ala Val Thr Leu Gly Lys Gly Val Glu Leu Val Lys Arg
   385 390 395 400
   Cys Arg Gln Thr Leu Ala Pro Glu Gly Ile Trp Ile Met Gly Ala Leu
   405 410 415
   Ala Ala Ile Ile Gly Leu Leu Val His Gly Met Val Asp Thr Val Trp
   420 425 430
   Tyr Arg Pro Pro Val Ser Thr Leu Trp Trp Leu Leu Val Ala Ile Val
   435 440 445
   Ala Ser Gln Trp Ala Ser Ala Gln Ala Arg Leu Glu Ala Ser Lys Glu
   450 455 460
   Glu Asn Glu Asp Lys Pro Leu Leu Ala Ser
   465 470
<210> 6
   <211> 475
   <212> PRT
   <213> Anabaena PCC7120
<400> 6
   Met Asn Leu Val Trp Gln Arg Phe Thr Leu Ser Ser Leu Pro Leu Lys
   1 5 10 15
   Gln Phe Leu Ala Thr Ser Tyr Leu His Arg Phe Leu Val Gly Leu Leu
   20 25 30
   Ser Ser Trp Arg Gln Thr Ser Phe Leu Leu Gln Trp Gly Asp Met Ile
   35 40 45
   Ala Ala Ala Leu Leu Ser Leu Ile Tyr Val Leu Ala Pro Phe Val Ser
   50 55 60
   Ser Thr Leu Val Gly Val Leu Leu Ile Ala Cys Val Gly Phe Trp Leu
   65                  70                  75                  80
   Leu Leu Thr Leu Ser Asp Glu Pro Ser Ser Asn Asn Asn Ser Leu Val
                   85                  90                  95
   Thr Pro Ile His Leu Leu Val Leu Leu Tyr Trp Gly Ile Ala Ala Val
               100                 105                 110
   Ala Thr Ala Leu Ser Pro Val Lys Lys Ala Ala Leu Thr Asp Leu Leu
           115                 120                 125
   Thr Leu Thr Leu Tyr Leu Leu Leu Phe Ala Leu Cys Ala Arg Val Leu
       130                 135                 140
   Arg Ser Pro Arg Leu Arg Ser Trp Ile Ile Thr Leu Tyr Leu Ser Ala
   145                 150                 155                 160
   Ser Leu Val Val Ser Ile Tyr Gly Met Arg Gln Trp Arg Phe Gly Ala
                   165                 170                 175
   Pro Pro Leu Ala Thr Trp Val Asp Pro Glu Ser Thr Leu Ser Lys Thr
               180                 185                 190
   Thr Arg Val Tyr Ser Tyr Leu Gly Asn Pro Asn Leu Leu Ala Gly Tyr
           195                 200                 205
   Leu Val Pro Ala Val Ile Phe Ser Leu Met Ala Val Phe Val Trp Gln
       210                 215                 220
   Gly Trp Ala Arg Lys Ser Leu Ala Val Thr Met Leu Phe Val Asn Thr
   225                 230                 235                 240
   Ala Cys Leu Ile Phe Thr Tyr Ser Arg Gly Gly Trp Ile Gly Leu Val
                   245                 250                 255
   Val Ala Val Leu Gly Ala Thr Ala Leu Leu Val Asp Trp Trp Ser Val
               260                 265                 270
   Gln Met Pro Pro Phe Trp Arg Thr Trp Ser Leu Pro Ile Leu Leu Gly
           275                 280                 285
   Gly Leu Ile Gly Val Leu Leu Ile Ala Val Leu Phe Val Glu Pro Val
       290                 295                 300
   Arg Phe Arg Val Leu Ser Ile Phe Ala Asp Arg Gln Asp Ser Ser Asn
   305                 310                 315                 320
   Asn Phe Arg Arg Asn Val Trp Asp Ala Val Phe Glu Met Ile Arg Asp
                   325                 330                 335
   Arg Pro Ile Ile Gly Ile Gly Pro Gly His Asn Ser Phe Asn Lys Val
               340                 345                 350
   Tyr Pro Leu Tyr Gln Arg Pro Arg Tyr Ser Ala Leu Ser Ala Tyr Ser
           355                 360                 365
   Ile Phe Leu Glu Val Ala Val Glu Met Gly Phe Val Gly Leu Ala Cys
       370                 375                 380
   Phe Leu Trp Leu Ile Ile Val Thr Ile Asn Thr Ala Phe Val Gln Leu
   385                 390                 395                 400
   Arg Gln Leu Arg Gln Ser Ala Asn Val Gln Gly Phe Trp Leu Val Gly
                   405                 410                 415
   Ala Leu Ala Thr Leu Leu Gly Met Leu Ala His Gly Thr Val Asp Thr
               420                 425                 430
   Ile Trp Phe Arg Pro Glu Val Asn Thr Leu Trp Trp Leu Met Val Ala
           435                 440                 445
   Leu Ile Ala Ser Tyr Trp Thr Pro Leu Ser Ala Asn Gln Cys Gln Glu
       450                 455                 460
   Leu Asn Leu Phe Lys Glu Glu Pro Thr Ser Asn
   465                 470                 475
<210> 7
   <211> 472
   <212> PRT
   <213> Nostoc punctiforme
<400> 7
   Met Asn Leu Val Trp Gln Leu Phe Thr Leu Ser Ser Leu Pro Leu Lys
   1 5 10 15
   Glu Tyr Leu Ala Thr Ser Tyr Val His Arg Ser Leu Val Gly Leu Leu
   20 25 30
   Ser Ser Trp Arg Gln Thr Ser Val Leu Ile Gln Trp Gly Asp Ala Ile
   35 40 45
   Ala Ala Val Leu Leu Ser Ser Ile Tyr Ala Leu Ala Pro Phe Ala Ser
   50 55 60
   Ser Thr Leu Val Gly Leu Leu Leu Val Ala Cys Val Gly Phe Trp Leu
   65 70 75 80
   Leu Leu Thr Leu Ser Asp Glu Val Thr Pro Ala Asn Val Ser Ser Val
   85 90 95
   Thr Pro Ile His Leu Leu Val Leu Leu Tyr Trp Gly Ile Ala Val Ile
   100 105 110
   Ala Thr Ala Leu Ser Pro Val Lys Lys Ala Ala Leu Asn Asp Leu Gly
   115 120 125
   Thr Leu Thr Leu Tyr Leu Leu Leu Phe Ala Leu Cys Ala Arg Val Leu
   130 135 140
   Arg Ser Pro Arg Leu Arg Ser Trp Ile Leu Thr Leu Tyr Leu His Val
   145 150 155 160
   Ser Leu Ile Val Ser Val Tyr Gly Leu Arg Gln Trp Phe Phe Gly Ala
   165 170 175
   Thr Ala Leu Ala Thr Trp Val Asp Pro Glu Ser Pro Leu Ser Lys Thr
   180 185 190
   Thr Arg Val Tyr Ser Tyr Leu Gly Asn Pro Asn Leu Leu Ala Gly Tyr
   195 200 205
   Leu Leu Pro Ala Val Ile Phe Ser Leu Val Ala Ile Phe Ala Trp Gln
   210 215 220
   Ser Trp Leu Lys Lys Ala Leu Ala Leu Thr Met Leu Ile Val Asn Thr
   225 230 235 240
   Ala Cys Leu Ile Leu Thr Phe Ser Arg Gly Gly Trp Ile Gly Leu Val
   245 250 255
   Val Ala Val Leu Ala Val Met Ala Leu Leu Val Phe Trp Lys Ser Val
   260 265 270
   Glu Met Pro Pro Phe Trp Arg Thr Trp Ser Leu Pro Ile Val Leu Gly
   275 280 285
   Gly Leu Ile Gly Ile Leu Leu Leu Ala Val Ile Phe Val Glu Pro Val
   290 295 300
   Arg Leu Arg Val Phe Ser Ile Phe Ala Asp Arg Gln Asp Ser Ser Asn
   305 310 315 320
   Asn Phe Arg Arg Asn Val Trp Asp Ala Val Phe Glu Met Ile Arg Asp
   325 330 335
   Arg Pro Ile Phe Gly Ile Gly Pro Gly His Asn Ser Phe Asn Lys Val
   340 345 350
   Tyr Pro Leu Tyr Gln His Pro Arg Tyr Thr Ala Leu Ser Ala Tyr Ser
   355 360 365
   Ile Leu Phe Glu Val Thr Val Glu Thr Gly Phe Val Gly Leu Ala Cys
   370 375 380
   Phe Leu Trp Leu Ile Ile Val Thr Phe Asn Thr Ala Leu Leu Gln Val
   385                 390                 395                 400
   Arg Arg Leu Arg Arg Leu Arg Ser Val Glu Gly Phe Trp Leu Ile Gly
                   405                 410                 415
   Ala Ile Ala Ile Leu Leu Gly Met Leu Ala His Gly Thr Val Asp Thr
               420                 425                 430
   Val Trp Tyr Arg Pro Glu Val Asn Thr Leu Trp Trp Leu Ile Val Ala
           435                 440                 445
   Leu Ile Ala Ser Tyr Trp Thr Pro Leu Thr Gln Asn Gln Thr Asn Pro
       450                 455                 460
   Ser Asn Pro Glu Pro Ala Val Asn
   465                 470
<210> 8
   <211> 1425
   <212> DNA
   <213> Anabaena PCC7120
<400> 8
   atgaatttag tctggcaacg atttacttta tcttctttac ctctaaaaca gtttctagct      60
   acaagttact tacatcggtt cctagtggga ctgttatctt cttggcggca aactagtttc     120
   ttacttcagt ggggagacat gattgcagct gcgttactca gcttgatata tgttttggct     180
   ccctttgtct ctagtactct cgttggtgtg ctgctgatag cttgtgtagg tttttggtta     240
   ttgttgactt tatctgatga accttcatca aacaataact cccttgttac tcccatacac     300
   ctgttggtgt tgctctattg gggaattgct gctgtagcaa cggcattatc accagtcaag     360
   aaggcagcat taactgattt gttaaccttg actttgtatt tgctactatt tgctctttgt     420
   gccagggtgc tgagatcgcc gcgtctgagg tcttggatca ttaccctcta cctatctgca     480
   tcactggttg tcagtatata tggaatgcga caatggcgtt ttggtgcgcc cccactggcg     540
   acttgggttg atccagagtc caccttgtct aaaaccacaa gggtttacag ttatttaggc     600
   aatcccaatt tgttggctgg ttatttagta ccggcggtga tttttagcct catggcagtt     660
   tttgtctggc agggctgggc aagaaaatct ttagctgtaa caatgctgtt tgtaaacact     720
   gcttgcctaa tttttactta tagtcgtggc ggctggattg gtcttgtggt agcagtctta     780
   ggggcgacgg cattgctagt tgattggtgg agtgtgcaaa tgccgccttt ttggcgaacc     840
   tggtcattac ccatactttt gggcggtttg atcggggtat tgttgattgc ggtgttattt     900
   gtcgagccag tccggtttcg agttctcagt atttttgccg atcgccaaga tagcagcaat     960
   aattttcgcc gcaacgtgtg ggatgctgtt tttgagatga tccgcgatcg cccaattatt    1020
   ggtattggcc ctggtcataa ttcttttaat aaagtctacc ctctttacca aagacctcgt    1080
   tatagtgctt taagtgccta ttccatcttc ctagaggtgg ctgtagaaat gggttttgtt    1140
   ggactagctt gctttctctg gttaattatc gtcactatta atacagcatt cgttcagcta    1200
   cgccaactgc gccaatctgc caatgtgcaa ggattttggt tggtgggtgc cttagccaca    1260
   ttgctgggaa tgctggctca cggtacggta gacactatat ggtttcgtcc ggaagttaat    1320
   actctttggt ggttaatggt tgctctcatt gctagctatt ggacaccttt atccgcaaac    1380
   caatgtcaag aactcaattt atttaaggaa gaacccacaa gcaac                    1425
<210> 9
   <211> 1419
   <212> DNA
   <213> Nostoc punctiforme
<400> 9
   atgaatttag tctggcaact atttacttta tcatctttac cgctcaaaga atatcttgct    60
   accagttacg tacaccgttc tctggtggga ctgttaagct cttggcggca aaccagcgtc   120
   ttgattcagt ggggagatgc gatagcagct gtattactca gctcaatata tgcccttgca   180
   ccttttgctt cgagtacttt ggtaggttta ttgctggtcg cttgtgtggg attttggcta   240
   ttgttgactt tatctgatga agtcacacca gcaaatgtct cgtcagtcac tcccattcat   300
   ctactggtat tgctctactg gggaattgcc gtaatcgcaa cagcattatc accagtgaaa   360
   aaagcggcac ttaacgactt gggaactttg accttgtatt tgctactatt tgccctttgt   420
   gccagggtat taaggtcgcc tcgcctccgg tcttggattc tcacccttta tctgcacgta   480
   tcgttaattg tcagtgtcta tggattgcgg caatggtttt ttggagccac agcactggca   540
   acttgggttg atccggaatc tcctctgtct aagactacaa gagtctacag ttatttagga   600
   aatcccaact tattggctgg atacctctta ccagcagtaa tttttagctt ggtggcaatt   660
   tttgcatggc aaagttggct caaaaaagcc ttagcattaa caatgttgat tgtcaatact   720
   gcctgcctga tcctgacttt tagtcgtggc ggttggattg gactagtggt ggcagttttg   780
   gcggtgatgg cattgctagt tttttggaag agtgtggaaa tgcctccttt ttggcgtact   840
   tggtcgctgc ccattgtctt aggaggttta attgggatat tactgttagc agtgatattt   900
   gtagagccag ttcgcctgcg ggtgttcagc atttttgctg accgtcaaga tagtagtaat   960
   aattttcgtc gaaatgtgtg ggatgctgtc tttgagatga ttcgcgatcg cccaattttc  1020
   ggtattggcc ctggtcacaa ctcttttaat aaagtttatc cgctctacca acaccctcgg  1080
   tacactgctt taagtgctta ttcgattttg tttgaagtga ctgtagaaac tgggtttgtt  1140
   ggtttagctt gctttctctg gctaataatc gtcacattta atacggcgct tttgcaagta  1200
   cgacgattgc gacgattgag aagtgtagag ggattttggt taattggagc gatcgctatt  1260
   ttgttgggta tgctcgctca cggcactgta gatactgtct ggtatcgtcc tgaagtcaat  1320
   accctctggt ggctcatcgt tgctttaatt gccagctact ggacaccttt aactcaaaac  1380
   cagacaaatc catctaaccc agaaccagca gtaaactaa                         1419

## Description

### FIELD AND BACKGROUND OF THE INVENTION

The present invention relates to plants characterized by enhanced growth and to methods and nucleic acid constructs useful for generating same.

Growth and productivity of crop plants are the main parameters of concern to a commercial grower. Such parameters are affected by numerous factors including the nature of the specific plant and allocation of resources within it, availability of resources in the growth environment and interactions with other organisms including pathogens.

Growth and productivity of most crop plants are limited by the availability of CO₂ to the carboxylating enzyme ribulose 1,5-bisphosphate carboxylase%xygenase (Rubisco). Such availability is determined by the ambient concentration of CO₂ and stomatal conductance, and the rate of CO₂ fixation by Rubisco as determined by the Km(CO₂) and Vmax of this enzyme [31-33].

In C3 plants, the concentration of CO₂ at the site of Rubisco is lower than the Km(CO₂) of the enzyme, particularly under water stress conditions. As such, these crop plants exhibit a substantial decrease in growth and productivity when exposed to low CO₂ conditions induced by, for example, stomatal closure which can be caused by water stress.

Many photosynthetic microorganisms are capable of concentrating CO₂ at the site of Rubisco to thereby overcome the limitation imposed by the low affinity of Rubisco for CO₂ [34].

Higher plants of the C4 and the CAM physiological groups can also raise the concentration of CO₂ at the site of Rubisco by means of dual carboxylations which are spatially (in C4) or timely (in CAM) separated.

Since plant growth and productivity especially in C3 crop plants are highly dependent on CO₂ availability to Rubisco and fixation rates, numerous attempts have been made to genetically modify plants in order to enhance CO₂ concentrations or fixation therein in hopes that such modification would lead to an increase in growth or yield.

As such, numerous studies attempted to introduce the CO₂ concentrating mechanisms of photosynthetic bacteria or C4 plants into C3 plants, so far with little or no success.

For example, studies attempting to genetically modify Rubisco in order to raise its affinity for CO₂ [35] and transformation of a C3 plant (rice) with several genes responsible for C4 metabolism have been described [36-40].

Although theoretically such approaches can lead to enhanced CO₂ fixation in C3 plants, results obtained from such studies have been disappointing.

There is thus a widely recognized need for, and it would be highly advantageous to have, a method of generating plants exhibiting enhanced growth and/or increased commercial yields.

### SUMMERY OF THE INVENTION

According to one aspect of the present invention there is provided method of enhancing growth and/or commercial yield of a plant, the method comprising expressing within the plant a polypeptide including an amino acid sequence at least 60 % homologous to that set forth in SEQ ID NOs:3, 5, 6 or 7 as characterized in the appended claims.

According to another aspect of the present invention there is provided a transformed plant expressing a polypeptide including an amino acid sequence at least 60 % homologous to that set forth in SEQ ID NOs:5,6 or 7 the transformed plant characterized by an enhanced growth as compared to similar non transformed plant grown under similar growth conditions.

According to further features in preferred embodiments of the invention described below, the plant is grown in an environment characterized by humidity lower than 40 %.

According to still further features in the described preferred embodiments the plant is grown in an environment characterized by a CO₂ concentration similar to or lower than in air, (approximately 0.035% CO₂ in air, and 10 micromolar CO₂ in solution).

According to still further features in the described preferred embodiments expressing the polypeptide within the plant is effected by transforming at least a portion of the plant cells with a nucleic acid construct including a polynucleotide region encoding the polypeptide.

According to still further features in the described preferred embodiments transforming is effected by a method selected from the group consisting of Agrobacterium mediated transformation, viral infection, electroporation and particle bombardment.

According to still further features in the described preferred embodiments the amino acid sequence is as set forth by SEQ ID NOs: 3, 5, 6, 7.

According to still further features in the described preferred embodiments the nucleic acid construct further includes a second polynucleotide region encoding a transit peptide.

According to still further features in the described preferred embodiments the nucleic acid construct further includes a promoter sequence for directing transcription of the first polynucleotide region.

According to still further features in the described preferred embodiments the nucleic acid construct further includes a promoter sequence for directing transcription of the first and the second polynucleotide regions.

According to still further features in the described preferred embodiments the promoter is functional in eukaryotic cells.

According to still further features in the described preferred embodiments the promoter is selected from the group consisting of a constitutive promoter, an inducible promoter, a developmentally regulated promoter and a tissue specific promoter.

According to still further features in the described preferred embodiments the plant is a C3 plant.

According to still further features in the described preferred embodiments the C3 plant is selected from the group consisting of tomato, soybean, potato, cucumber, cotton, wheat, rice, barley, sunflower, banana, tobacco, lettuce, cabbage, petunia, solidago and poplar. According to still further features in the described preferred embodiments the plant is a C4 plant.

According to still further features in the described preferred embodiments the C4 plant is selected from the group consisting of corn, sugar cane and sohrgum.

According to still further features in the described preferred embodiments the plant expressing the polypeptide is characterized by a growth rate which is at least 10 % higher than that of a similar plant not expressing the polypeptide when both are grown under similar growth conditions where CO₂ becomes limiting.

According to still further features in the described preferred embodiments the growth rate is determined by at least one growth parameter selected from the group consisting of increased fresh weight, increased dry weight, increased root growth, increased shoot growth and flower development over time.

According to still further features in the described preferred embodiments the transformed plant is further characterized by an increased commercial yield as compared to similar non transformed plant grown under similar CO₂ limiting conditions.

According to yet another aspect of the present invention there is provided a nucleic acid expression construct comprising: (a) a first polynucleotide region encoding a polypeptide including an amino acid sequences at least 60 % homologous to that set forth by SEQ ID NOs:5, 6 or 7; and (b) a second polynucleotide region functional as a promoter and being for directing the transcription of the first polynucleotide region in eukaryotic cells.

According to still further features in the described preferred embodiments the promoter is selected from the group consisting of a constitutive promoter, an inducible prompter and a tissue specific promoter.

According to still further features in the described preferred embodiments the promoter is a plant promoter.

According to still further features in the described preferred embodiments the first polynucleotide region further encodes a transit peptide being translationally fused to the polypeptide.

The present invention successfully addresses the shortcomings of the presently known configurations by providing plants characterized by enhanced growth and to methods and nucleic acid constructs useful for generating same.

### BRIEF DESCRIPTION OF THE DRAWINGS

The invention is herein described, by way of example only, with reference to the accompanying drawings. Width specific reference now to the drawings in detail, it is stressed that the particular shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

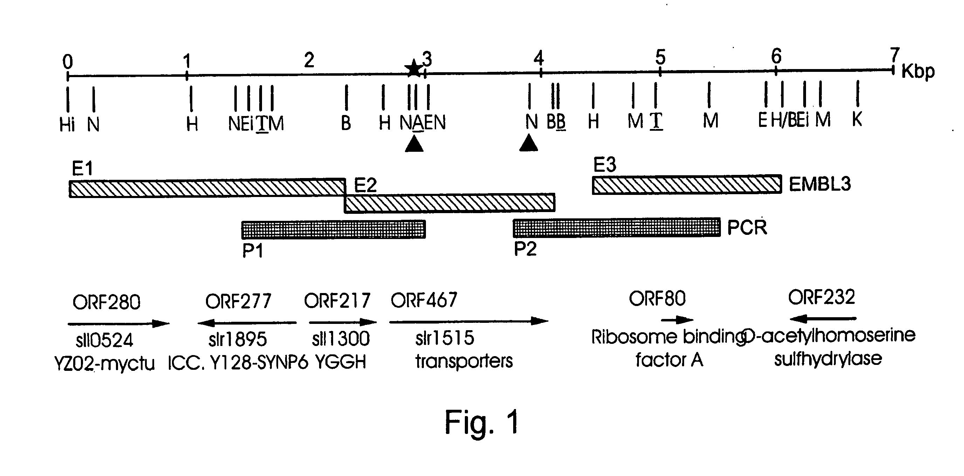

FIG. 1 is a schematic representation of a genomic region in *Synechococcus* sp. PCC 7942 where an insertion (indicated by a star) of an inactivation library fragment led to the formation of mutant IL-2. DNA sequence is available in the GenBank, Accession number U62616. Restriction sites are marked as: A - *Apa*I*,* B *- Bam*HI, Ei *- Eco*RI*,* E - *Eco*RV, *H - Hinc*II*,* Hi *- Hind*III*, K - Kpn*I*, M - Mfe*I*,* N *- NheI,* T *- Taq*I*.* Underlined letters represent the terminate position of the DNA fragments that were used as probes. Relevant fragments isolated from an EMBL3 library are marked E1, E2 and E3. P1 and P2 are fragments obtained by PCR. Triangles indicate sites where a cartridge encoding Kan^{r} was inserted. Open reading frames are marked by an arrow and their similarities to other proteins are noted. Sll and slr (followed by four digits) are the homologous genes in *Synechocystis* sp. PCC 6803 [23]; YZ02-myctu, Accession No. Q10536; ICC, Accession No. P36650; Y128-SYNP6, Accession No. P05677; YGGH, Accession No. P44648; Ribosome binding factor A homologous to s110754 and to P45141; O-acetylhomoserine sulfhydrylase homologous to s110077 and NifS. ORF280 started upstream of the schematic representation presented herein.

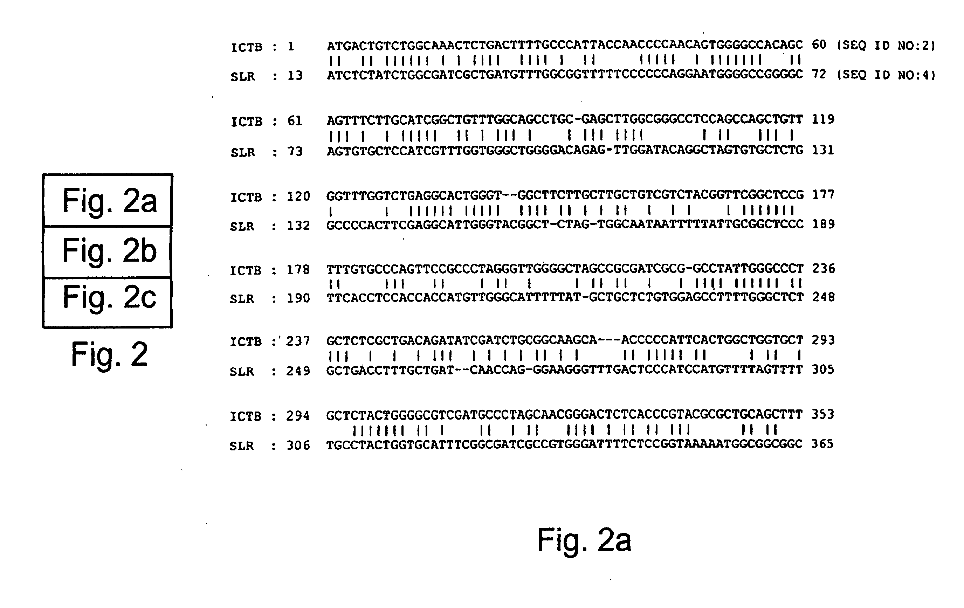

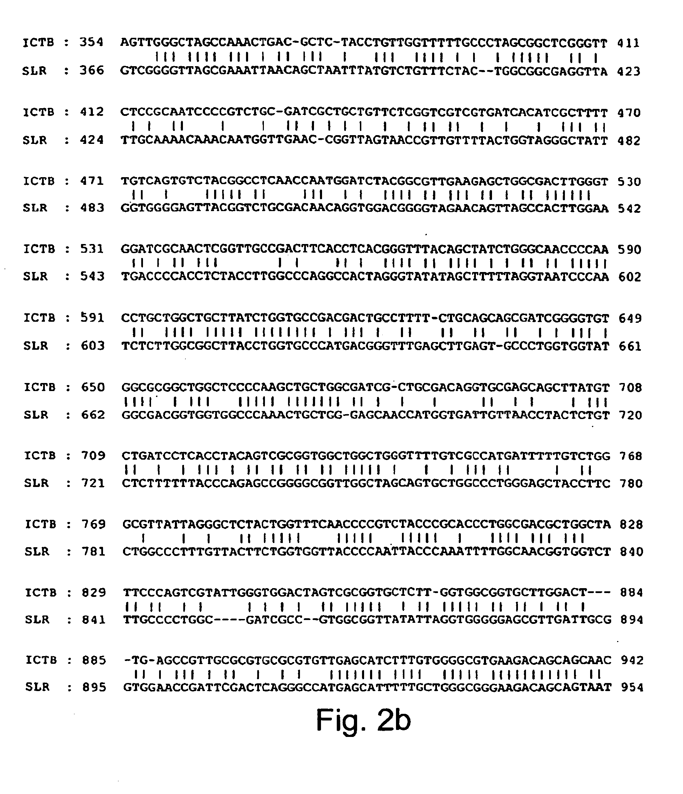

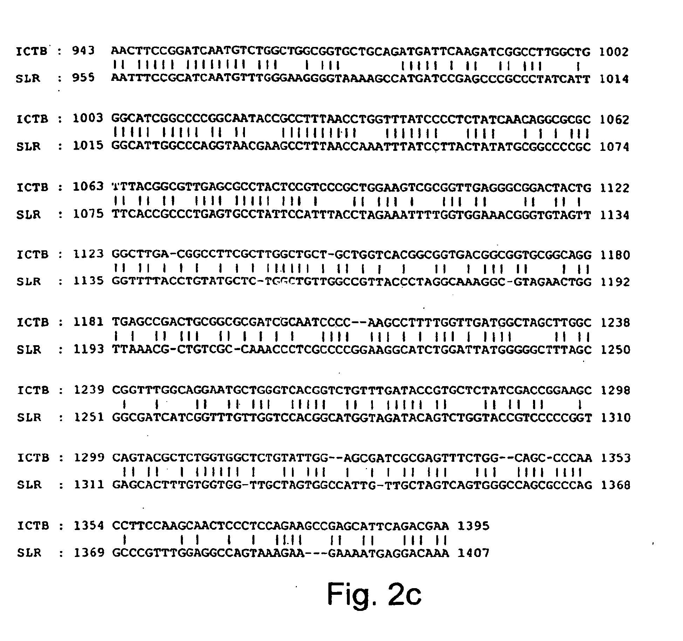

FIG. 2 shows nucleic acid sequence alignment between ORF467 (ICTB, SEQ ID NO:2) and sIr1515 (SLR, SEQ ID NO:4). Vertical lines indicate nucleotide identity. Gaps are indicated by hyphens. Alignment was performed using the Blast software where gap penalty equals 10 for existence and 10 for extension, average match equals 10 and average mismatch equals -5. Identical nucleotides equals 56 %.

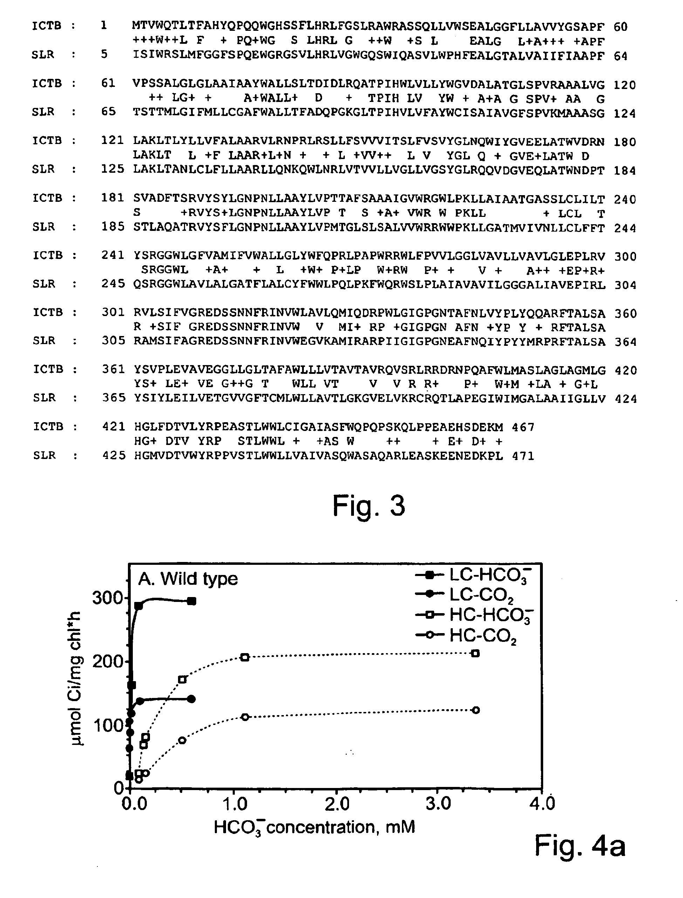

FIG. 3 shows amino acid sequence alignment between the IctB protein (ICTB, SEQ ID NO:3) and the protein encoded by sIr1515 (SLR, SEQ ID NO:5). Identical amino acids are marked by their single letter code between the aligned sequences, similar amino acids are indicated by a plus sign. Alignment was performed using the Blast software where gap open penalty equals 11, gap extension penalty equals 1 and matrix is blosum62. Identical amino acids equals 47 %, similar amino acids equals 16 %, total homology equals 63 %.
FIGs. 4a-b are graphs showing the rates of CO₂ and of HCO₃⁻ uptake by *Synechococcus* PCC 7942 (4a) and mutant IL-2 (4b) as a function of external Ci concentration. LC and HC are cells grown under low (air) or high CO₂ (5%CO₂ in air), respectively. The rates were assessed from measurements during steady state photosynthesis using a membrane inlet mass spectrometer (MIMS) [6, 7, 22].

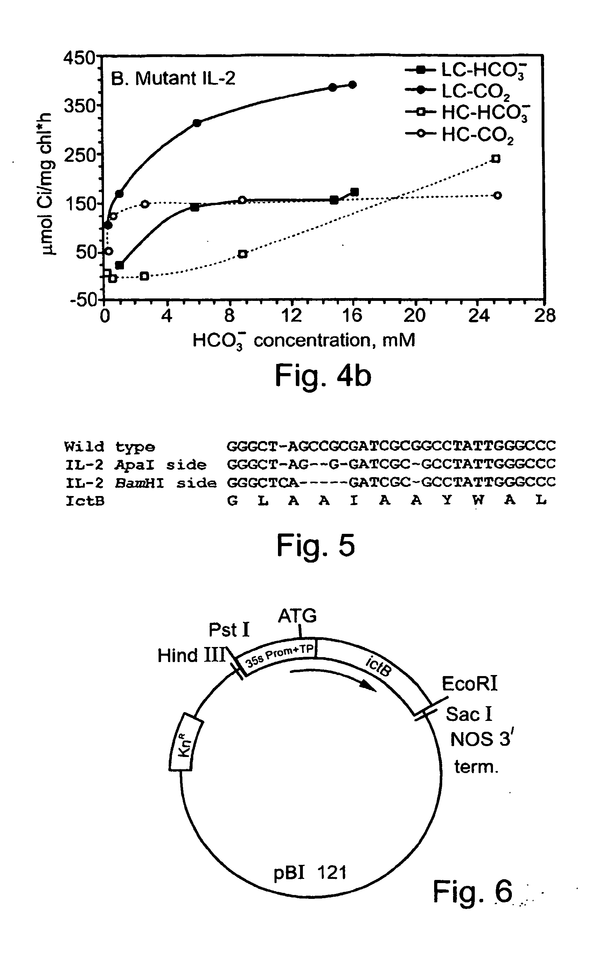

FIG. 5 presents DNA sequence homology comparison of a region of *ictB* found in *Synechococcus* PCC 7942 and in mutant IL-2. This region was duplicated in the mutant due to a single cross-over event. Compared with the wild type, one additional nucleotide and a deletion of six nucleotides were found in the *Bam*HI side, and 4 nucleotides were deleted in the *Apa*I side (see Figure 1). These changes resulted in stop codons in *IctB* after 168 or 80 amino acids in the *Bam*HI and *Apo*I sides, respectively. The sequence shown by this Figure starts from the 69^{th} amino acid of *ictB.*
FIG. 6 illustrates the *ictB* construct used in generating the transgenic plants of the present invention, including a 35S promoter, the transit peptide (TP) from the small subunit of pea Rubisco (nucleotide coordinates 329-498 of GeneBank Accession number x04334 where we replaced the G in position 498 with a T, the *ictB* coding region, the NOS termination and kanamycin-resistance (Kn^{R}) within the binary vector pBI121 from Clontech.

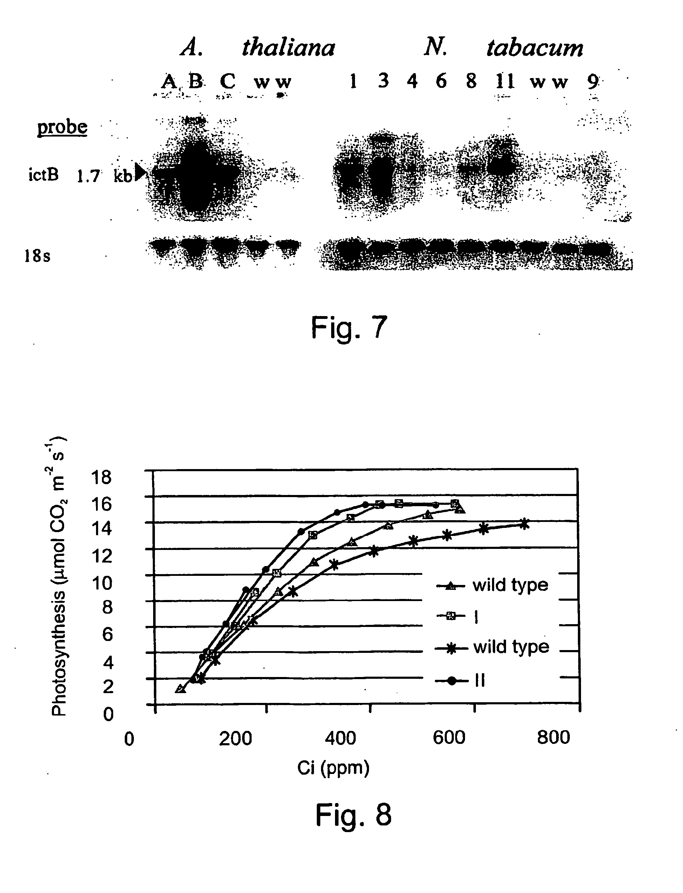

FIG. 7 is a Northern blot analysis of transgenic and wild type (w) *Arabidopsis* and tobacco plants using both *ictB* and 18S rDNA as probes.
FIG. 8 illustrates the rate of photosynthesis as affected by the intercellular concentration of CO₂ in wild type and the transgenic tobacco plants of the present invention; plants 1 and 11 are transgenic.

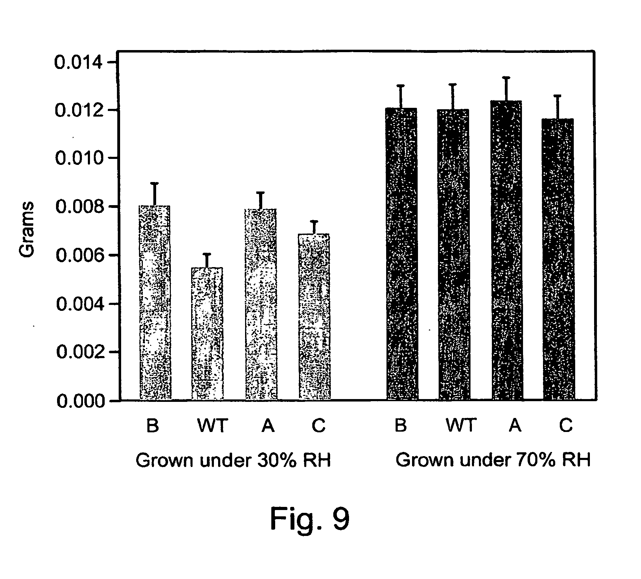

FIG. 9 illustrates growth experiments conducted on both transgenic (A, B and C) and wild type (WT) *Arabidopsis* plants. Each growth pot included one wild type and three transgenic plants. Data are provided as the average dry weight of the plants +/- S.D. Growth conditions are described in the Examples section.

### DESCRIPTION OF THE PREFERRED EMBODIMENTS

The present invention is of a method of generating plants characterized by enhanced growth and/or fruit yield and/or flowering rate, of plants generated thereby, and of nucleic acid constructs utilized by such a method. Specifically, the present invention can be used to substantially increase the growth rate and/or fruit, yield of C3 plants especially when grown under conditions characterized by low humidity and/or a low CO₂ concentration.

The principles and operation of the present invention may be better understood with reference to the drawings and accompanying descriptions.

The invention is capable of other embodiments or of being practiced or carried out in various ways. Also, it is to be understood that the phraseology and terminology employed herein is for the purpose of description and should not be regarded as limiting.

Increasing the growth size/rate and/or commercial yield of crop plants is of paramount importance especially in regions in which growth/cultivation conditions are suboptimal due to a lack of, for example, water.

While reducing the present invention to practice the inventors have discovered that plants expressing exogenous polynucleotides encoding a putative cyanobacterial inorganic carbon transporter are characterized by enhanced growth, especially when grown under conditions characterized by low humidity or low CO₂ concentrations.

Thus, according to the present invention there is provided a transformed plant expressing a polypeptide including an amino acid sequence which is at least 60 % homologous to that set forth in SEQ ID NO: 5, 6 or 7.

The transformed plant of the present invention is characterized by enhanced growth as compared to similar non transformed plant grown under similar growth conditions.

As used herein, the phrase "enhanced growth" refers to an enhanced growth rate, or to an increased growth size/weight of the whole plant or preferably the commercial portion of the plant (increased yield) as determined by fresh weight, dry weight or size of the plant or commercial portion thereof.

As is further detailed in the Examples section which follows, the transformed plants of the present invention exhibit, for example, a growth rate which is at least 10 % higher than that of a similar non transformed plant when both plants are grown under similar growth conditions.

According to a preferred embodiment of the present invention, the polypeptide is at least 60 %, preferably at least 65 %, more preferably at least 70 %, still more preferably at least 75 %, yet more preferably at least 80 %, more preferably at least 85 %, more preferably at least 90 %, yet more preferably at least 95 %, ideally 95-100 % homologous (identical + similar) to SEQ ID NO: 3, 5, 6 or 7 or a portion thereof as determined using the Blast software where gap open penalty equals 11, gap extension, penalty equals 1 and matrix is blosum62.

According to preferred embodiments of the present invention, the growth conditions are characterized by humidity of less than 40 % and/or CO₂ concentration which is lower than in air.

The transformed plant of the present invention can be any plant including, but not limited to, C3 plants such as, for example, tomato, soybean, potato, cucumber, cotton, wheat, rice, barley or C4 plants, such as, for example, corn, sugar cane, sohrgum and others.

The transformed plant of the present invention is generated by introducing a nucleic acid molecule or polynucleotide encoding the polypeptide(s) described above into cells of the plant.

Such a nucleic acid molecule or polynucleotide can have a sequence corresponding to at least a portion of SEQ ID NO:2, 4, 8 or 9 the portion encoding a polypeptide contributing the increased growth trait.

Alternatively or additionally the nucleic acid molecule can have a sequence which is at least 60 %, preferably at least 65 %, more preferably at least 70 %, still more preferably at least 75 %, yet more preferably at least 80 %, more preferably at least 85 %, more preferably at least 90 %, yet more preferably at least 95 %, ideally 95-100 % identical to that portion, as determined using the Blast software where gap penalty equals 10 for existence and 10 for extension, average match equals 10 and average mismatch equals -5. It will be appreciated in this respect that SEQ ID NO:2, 4, 8 or 9 can be readily used to isolate homologous sequences which can be tested as described in the Examples section that follows for their bicarbonate transport activity. Methods for isolating such homologous sequences are extensively described in, for example, Sambrook *et al.* [9] and may include hybridization and PCR amplification.

Still alternatively or additionally the nucleic acid molecule can have a sequence capable of hybridizing with the portion of SEQ ID NO:2, 4, 8 or 9. Hybridization for long nucleic acids (e.g., above 200 bp in length) is effected according to preferred embodiments of the present invention by stringent or moderate hybridization, wherein stringent hybridization is effected by a hybridization solution containing 10 % dextrane sulfate, 1 M NaCl, I % SDS and 5x10⁶ cpm ³²p labeled probe, at 65°C, with a final wash solution of 0.2 x SSC and 0.1 % SDS and final wash at 65°C; whereas moderate hybridization is effected by a hybridization solution containing 10 % dextrane sulfate, 1 M NaCl, 1 % SDS and 5 x 10⁶ cpm ³²p labeled probe, at 65 °C, with a final wash solution of 1 x SSC and 0.1 % SDS and final wash at 50 °C.

Preferably, the polypeptide encoded by the nucleic acid molecule of the present invention includes an N terminal transit peptide fused thereto which serves for directing the polypeptide to a specific membrane. Such a membrane can be, for example, the cell membrane, wherein the polypeptide will serve to transport bicarbonate from the apoplast into the cytoplasm, or, such a membrane can be the outer and preferably the inner chloroplast membrane. Transit peptides which function as herein described are well known in the art. Further description of such transit peptides is found in, for example, Johnson et al. The Plant Cell (1990) 2:525-532; Sauer et al. EMBO J. (1990) 9:3045-3050; Mueckler et al. Science (1985) 229:941-945; Von Heijne, Eur. J. Biochem. (1983) 133:17-21; Yon Heijne, J. Mol. Biol. (1986) 189:239-242; Iturriaga et al. The Plant Cell (1989) 1:381-390; McKnight et al., Nucl. Acid Res. (1990) 18:4939-4943; Matsuoka and Nakamura, Proc. Natl. Acad. Sci. USA (1991) 88:834-838. A recent text book entitled "Recombinant proteins from plants", Eds. C. Cunningham and A.J.R. Porter, 1998 Humana Press Totowa, N.J. describe methods for the production of recombinant proteins in plants and methods for targeting the proteins to different compartments in the plant cell. The book by Cunningham and Porter is incorporated herein by reference. It will however be appreciated by one of skills in the art that a large number of membrane integrated proteins fail to poses a removable transit peptide. It is accepted that in such cases a certain amino acid sequence in said proteins serves not only as a structural portion of the protein, but also as a transit peptide.

Preferably, the nucleic acid molecule of the present invention is included within a nucleic acid construct designed as a vector for transforming plant cells thereby enabling expression of the nucleic acid molecule within such cells.

Plant expression can be effected by introducing the nucleic acid molecule of the present invention (preferably using the nucleic acid construct) downstream of a plant promoter present in endogenous genomic or organelle polynucleotide sequences (e.g., chloroplast or mitochondria), thereby enabling expression thereof within the plant cells.

In such cases, the nucleic acid construct further includes sequences which enable to "knock-in" the nucleic acid molecule into specific or random polynucleotide regions of such genomic or organelle polynucleotide sequences.

Preferably, the nucleic acid construct of the present invention further includes a plant promoter which serves for directing expression of the nucleic acid molecule within plant cells.

As used herein in the specification and in the claims section that follows the phrase "plant promoter" includes a promoter which can direct gene expression in plant cells (including DNA containing organelles). Such a promoter can be derived from a plant, bacterial, viral, fungal or animal origin. Such a promoter can be constitutive, i.e., capable of directing high level of gene expression in a plurality of plant tissues, tissue specific, i.e., capable of directing gene expression in a particular plant tissue or tissues, inducible, i.e., capable of directing gene expression under a stimulus, or chimeric.

Thus, the plant promoter employed can be a constitutive promoter, a tissue specific promoter, an inducible promoter or a chimeric promoter.

Examples of constitutive plant promoters include, without limitation, CaMV35S and CaMV19S promoters, FMV34S promoter, sugarcane bacilliform badnavirus promoter, CsVMV promoter, *Arabidopsis* ACT2/ACT8 actin promoter, *Arabidopsis* ubiquitin UBQ1 promoter, barley leaf thionin BTH6 promoter, and rice actin promoter.

Examples of tissue specific promoters include, without being limited to, bean phaseolin storage protein promoter, DLEC promoter, PHSβ promoter, zein storage protein promoter, conglutin gamma promoter from soybean, AT2S1 gene promoter, ACT11 actin promoter from *Arabidopsis,* napA promoter from *Brassica napus* and potato patatin gene promoter.

The inducible promoter is a promoter induced by a specific stimuli such as stress conditions comprising, for example, light, temperature, chemicals, drought, high salinity, osmotic shock, oxidant conditions or in case of pathogenicity and include, without being limited to, the light-inducible promoter derived from the pea rbcS gene, the promoter from the alfalfa rbcS gene, the promoters DRE, MYC and MYB active in drought; the promoters INT, INPS, prxEa, Ha hsp 17.7G4 and RD21 active in high salinity and osmotic stress, and the promoters hsr203J and str246C active in pathogenic stress.

The nucleic acid construct of the present invention preferably further includes additional polynucleotide regions which provide a broad host range prokaryote replication origin; a prokaryote selectable marker; and, for *Agrobacterium* transformations, T DNA sequences for *Agrobacterium-mediated* transfer to plant chromosomes. Where the heterologous sequence is not readily amenable to detection, the construct will preferably also have a selectable marker gene suitable for determining if a plant cell has been transformed. A general review of suitable markers for the members of the grass family is found in Wilmink and Dons, Plant Mol. Biol. Reptr. (1993) 11:165-185.

Suitable prokaryote selectable markers include resistance toward antibiotics such as ampicillin, kanamycin or tetracycline. Other DNA sequences encoding additional functions may also be present in the vector, as is known in the art.

Sequences suitable for permitting integration of the heterologous sequence into the plant genome are also recommended. These might include transposon sequences as well as Ti sequences which permit random insertion of a heterologous expression cassette into a plant genome.

The nucleic acid construct of the present invention can be utilized to stably or transiently transform plant cells. In stable transformation, the nucleic acid molecule of the present invention is integrated into the plant genome and as such it represents a stable and inherited trait. In transient transformation, the nucleic acid molecule is expressed by the cell transformed but it is not integrated into the genome and as such it represents a transient trait.

There are various methods of introducing foreign genes into both monocotyledonous and dicotyledonous plants (Potrykus, I., Annu. Rev. Plant. Physiol., Plant. Mol. Biol. (1991) 42:205-225; Shimamoto et al., Nature (1989) 338:274-276).

The principle methods of causing stable integration of exogenous DNA into plant genomic DNA include two main approaches:
(i) *Agrobacterium-*mediated gene transfer: Klee et al. (1987) Annu. Rev. Plant Physiol. 38:467-486; Klee androgens in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Arntzen, C. J., Butterworth Publishers, Boston, Mass. (1989) p. 93-112.
(ii) direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The *Agrobacterium* system includes the use of plasmid vectors that contain defined DNA segments that integrate into the plant genomic DNA. Methods of inoculation of the plant tissue vary depending upon the plant species and the *Agrobacterium* delivery system. A widely used approach is the leaf disc procedure which can be performed with any tissue explant that provides a good source for initiation of whole plant differentiation. Horsch et al. in Plant Molecular Biology Manual A5, Kluwer Academic Publishers, Dordrecht (1988) p. 1-9. A supplementary approach employs the *Agrobacterium* delivery system in combination with vacuum infiltration. The *Agrobacterium* system is especially viable in the creation of transgenic dicotyledenous plants.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Following stable transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Micropropagation is a process of growing new generation plants from a single piece of tissue that has been excised from a selected parent plant or cultivar. This process permits the mass reproduction of plants having the preferred tissue expressing the fusion protein. The new generation plants which are produced are genetically identical to, and have all of the characteristics of, the original plant. Micropropagation allows mass production of quality plant material in a short period of time and offers a rapid multiplication of selected cultivars in the preservation of the characteristics of the original transgenic or transformed plant. The advantages of cloning plants are the speed of plant multiplication and the quality and uniformity of plants produced.

Micropropagation is a multi-stage procedure that requires alteration of culture medium or growth conditions between stages. Thus, the micropropagation process involves four basic stages: Stage one, initial tissue culturing; stage two, tissue culture multiplication; stage three, differentiation and plant formation; and stage four, greenhouse culturing and hardening. During stage one, initial tissue culturing, the tissue culture is established and certified contaminant-free. During stage two, the initial tissue culture is multiplied until a sufficient number of tissue samples are produced to meet production goals. During stage three, the tissue samples grown in stage two are divided and grown into individual plantlets. At stage four, the transformed plantlets are transferred to a greenhouse for hardening where the plants' tolerance to light is gradually increased so that it can be grown in the natural environment.

Although stable transformation is presently preferred, transient transformation of leaf cells, meristematic cells or the whole plant is also envisaged by the present invention.

Transient transformation can be effected by any of the direct DNA transfer methods described above or by viral infection using modified plant viruses.

Viruses that have been shown to be useful for the transformation of plant hosts include CaMV, TMV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Construction of plant RNA viruses for the introduction and expression of non-viral exogenous nucleic acid sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; and Takamatsu et al. FEBS Letters (1990) 269:73-76.

When the virus is a DNA virus, suitable modifications can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous nucleic acid sequences such as those included in the construct of the present invention is demonstrated by the above references as well as in U.S. Pat. No. 5,316,931.

In one embodiment, a plant viral nucleic acid is provided in which the native coat protein coding sequence has been deleted from a viral nucleic acid, a non-native plant viral coat protein coding sequence and a non-native promoter, preferably the subgenomic promoter of the non-native coat protein coding sequence, capable of expression in the plant host, packaging of the recombinant plant viral nucleic acid, and ensuring a systemic infection of the host by the recombinant plant viral nucleic acid, has been inserted. Alternatively, the coat protein gene may be inactivated by insertion of the non-native nucleic acid sequence within it, such that a protein is produced. The recombinant plant viral nucleic acid may contain one or more additional non-native subgenomic promoters. Each non-native subgenomic promoter is capable of transcribing or expressing adjacent genes or nucleic acid sequences in the plant host and incapable of recombination with each other and with native subgenomic promoters. Non-native (foreign) nucleic acid sequences may be inserted adjacent the native plant viral subgenomic promoter or the native and a non-native plant viral subgenomic promoters if more than one nucleic acid sequence is included. The non-native nucleic acid sequences are transcribed or expressed in the host plant under control of the subgenomic promoter to produce the desired products.

In a second embodiment, a recombinant plant viral nucleic acid is provided as in the first embodiment except that the native coat protein coding sequence is placed adjacent one of the non-native coat protein subgenomic promoters instead of a non-native coat protein coding sequence.

In a third embodiment, a recombinant plant viral nucleic acid is provided in which the native coat protein gene is adjacent its subgenomic promoter and one or more non-native subgenomic promoters have been inserted into the viral nucleic acid. The inserted non-native subgenomic promoters are capable of transcribing or expressing adjacent genes in a plant host and are incapable of recombination with each other and with native subgenomic promoters. Non-native nucleic acid sequences may be inserted adjacent the non-native subgenomic plant viral promoters such that said sequences are transcribed or expressed in the host plant under control of the subgenomic promoters to produce the desired product.

In a fourth embodiment, a recombinant plant viral nucleic acid is provided as in the third embodiment except that the native coat protein coding sequence is replaced by a non-native coat protein coding sequence.

The viral vectors are encapsidated by the coat proteins encoded by the recombinant plant viral nucleic acid to produce a recombinant plant virus. The recombinant plant viral nucleic acid or recombinant plant virus is used to infect appropriate host plants. The recombinant plant viral nucleic acid is capable of replication in the host, systemic spread in the host, and transcription or expression of foreign gene(s) (isolated nucleic acid) in the host to produce the desired protein.

In addition to the above, the nucleic acid molecule of the present invention can also be introduced into a chloroplast genome thereby enabling chloroplast expression.

A technique for introducing exogenous nucleic acid sequences to the genome of the chloroplasts is known. This technique involves the following procedures. First, plant cells are chemically treated so as to reduce the number of chloroplasts per cell to about one. Then, the exogenous nucleic acid is introduced via particle bombardment into the cells with the aim of introducing at least one exogenous nucleic acid molecule into the chloroplasts. The exogenous nucleic acid is selected such that it is integratable into the chloroplast's genome via homologous recombination which is readily effected by enzymes inherent to the chloroplast. To this end, the exogenous nucleic acid includes, in addition to a gene of interest, at least one nucleic acid stretch which is derived from the chloroplast's genome. In addition, the exogenous nucleic acid includes a selectable marker, which serves by sequential selection procedures to ascertain that all or substantially all of the copies of the chloroplast genomes following such selection will include the exogenous nucleic acid. Further details relating to this technique are found in U.S. Pat. Nos. 4,945,050; and 5,693,507 which are incorporated herein by reference. A polypeptide can thus be produced by the protein expression system of the chloroplast and become integrated into the chloroplast's inner membrane.

Thus, the present invention provides methods, nucleic acid constructs and transformed plants generated using such methods and constructs, which transformed plants are characterized by an enhanced growth rate and/or increased commercial yield.

Additional objects, advantages, and novel features of the present invention will become apparent to one ordinarily skilled in the art upon examination of the following examples; which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### EXAMPLE 1

### ictB isolation and characterization Materials and Experimental Methods

### Growth conditions:

Cultures of *Synechococcus* sp. strain PCC 7942 and mutant IL.-2 thereof were grown at 30 °C in BG₁₁ medium supplemented with 20 mM Hepes-NaOH pH 7.8 and 25 µg mL⁻¹ kanamycin (in the case of the mutant). The medium was aerated with either 5 .% v/v CO₂ in air (high CO₂) or 0.0175 % v/v CO₂ in air (low CO₂) which was prepared by mixing air with CO₂-free air at a 1:1 ratio. *Escherichia coli* (strain DH5o) were grown on an LB medium [9] supplemented with either kanamycin (50 µg/mL) or ampicillin (50 µg/mL) when required.

### Measurements of photosynthesis and Ci uptake:

The rates of inorganic carbon (Ci)-dependent O₂ evolution were measured by an O₂ electrode as described elsewhere [10] and by a membrane inlet mass spectrometer (MIMS, [6, 11]). The MIMS was also used for assessments of CO₂ and HCO₃⁻ uptake during steady state photosynthesis [6]. Ci flukes following supply of CO₂ or HCO₃⁻ were determined by the filtering centrifugation technique [10]. High-CO₂ grown cells in the log phase of growth were transferred to either low or high CO₂ 12 hours before conducting the experiments. Following harvest, the cells were resuspended in 25 mM Hepes-NaOH pH 8.0 and aerated with air (Ci concentration was about 0.4 mM) under light flux of 100 µmol photon quanta m⁻² s⁻¹. Aliquots were withdrawn, immediately placed in microfuge tubes and kept under similar light and temperature conditions. Small amounts of ¹⁴C-CO₂ or ¹⁴C-HCO₃⁻ which did not affect the final Ci concentration, were injected, and the Ci uptake terminated after 5 seconds by centrifugation.

### General DNA manipulations:

Genomic DNA was isolated as described elsewhere [12]. Standard recombinant DNA techniques were used for cloning and Southern analyses [12-13] using the Random Primed DNA Labeling Kit or the DIG system (Boehringer, Mannheim). Sequence analysis was performed using the Dye Terminator cycle sequencing kit, ABI Prism (377 DNA sequencing Perkin Elmer). The genomic library used herein was constructed using a Lambda EMBL3/BamHI vector kit available from Stratagene (La Jolla, CA).

### Construction and isolation of mutant IL-2:

A modification of the method developed by Dolganov and Grossman [14] was used to raise and isolate new high-CO₂-requiring mutants [4, 5]. Briefly, genomic DNA was digested with *Taq*I and ligated into the *Acc*I site of the polylinker of a modified Bluescript SK plasmid. The bluescript borne gene for conferring ampicillin resistance was inactivated by the insertion of a cartridge encoding kanamycin resistance (Kan^{r}, [8]) (within the ScaI site). *Synechococcus* sp. strain PCC 7942 cells were transfected with the library [12]. Single crossover events which conferred Kan^{r} led to inactivation of various genes. The Kan^{r} cells were exposed to low CO₂ conditions for 8 hours for adaptation, followed by an ampicillin treatment (400 µg/mL) for 12 hours. Cells capable of adapting to low CO₂ and thus able to grow under these conditions were eliminated by this treatment. The high-CO₂-requiring mutant, IL-2, unable to divide under low CO₂ conditions, survived, and was rescued following the removal of ampicillin and growth in the presence of high CO₂ concentration.

### Cloning of the relevant impaired genomic region from mutant IL-2:

DNA isolated from the mutant was digested with *Apa*I located on one side of the *Acc*I site in the polylinker; with *Bam*HI or *Eco*RI*,* located on the other side of the *Acc*I site; or with *Mfe*I that does not cleave the vector or the Kan^{r} cartridge. These enzymes also cleaved the genomic DNA. The digested DNA was self-ligated followed by transfection of competent *E. coli* cells (strain DH5α). Kan^{r} colonies carrying the vector sequences bearing the origin of replication, the Kan^{r} cartridge and part of the inactivated gene were then isolated. This procedure was used to clone the flanking regions on both sides of the vector inserted into the mutant. A 1.3 Kbp *Apa*I and a 0.8 Kbp *Bam*HI fragments isolated from the plasmids (one *Apa*I site and *Bam*HI site originated from the vector's polylinker) were used as probes to identify the relevant clones in an EMBL3 genomic library of a wild type genome, and for Southern analyses. The location of these fragments in the wild type genome (SEQ ID NO:1) is schematically shown in Figure 1. The *Apa*I fragment is between positions 1600 to 2899 (of SEQ ID NO:1), marked as T and A in Figure 1; the *Bam*HI fragment is between positions 4125 to 4957 (of SEQ ID NO:1) marked as B and T in Figure 1. The 0.8 Kbp *Bam*HI fragment hybridized with the 1.6 Kbp *Hinc*II fragment (marked E3 in Figure 1). The 1.3 Kbp *Apa*I fragment hybridized with an *Eco*RI fragment of about 6 Kbp. Interestingly, this fragment could not be cloned from the genomic library into *E. coli.* Therefore, the *Bam*HI site was used (position 2348, SEQ ID NO:1, Figure 1) to split the EMBL3 clone into two clonable fragments of 4.0 and 1.8 Kbp (E1 and E2, respectively, E1 starts from a *Sau*3A site upstream of the *Hind*III site positioned at the beginning of Figure 1). Confirmation that these three fragments were indeed located as shown in Figure 1 was obtained by PCR using wild type DNA as template, leading to the synthesis of fragments P1 and P2 (Figure 1). Sequence analyses enabled comparison of the relevant region in IL-2 with the corresponding sequence in the wild-type.

### Physiological analysis of the IL-2 mutants:

The IL-2 mutant grew nearly the same as the wild type cells in the presence of high CO₂ concentration but was unable to grow under low CO₂. Analysis of the photosynthetic rate as a function of external Ci concentration revealed that the apparent photosynthetic affinity of the IL-2 mutant was 20 mM Ci, which is about 100 times higher than the concentration of Ci at the low CO₂ conditions. The curves relating to the photosynthetic rate as a function of Ci concentration, in IL-2, were similar to those obtained with other high-CO₂-requiring mutants of *Synechococcus* PCC 7942 [16, 17]. These data suggested that the inability of IL-2 to grow under low CO₂ is due to the poor photosynthetic performance of this mutant.

High-CO₂-requiring mutants showing such characteristics were recognized among mutants bearing aberrant carboxysomes [9, 10, 12, 18, 19] or defective in energization of Ci uptake [20, 21]. All the carboxysome-defective mutants characterized to date were able to accumulate Ci within the cells similarly to wild type cells. However, they were unable to utilize it efficiently in photosynthesis due to low activation state of rubisco in mutant cells exposed to low CO₂ [10]. This was not the case for mutant IL-2 which possessed normal carboxysomes but exhibited impaired HCO₃⁻ uptake (Table 1, Figures 4a-b). Measurements of ¹⁴Ci accumulation indicated that HCO₃ and CO₂ uptake were similar in the high-CO₂-grown wild type and the mutant (Table 1).

**Table 1**

| | **CO₂ Uptake** | | **HCO₃⁻ Uptake** | |
|---|---|---|---|---|
| | **High CO₂** | **Low CO₂** | **High CO₂** | **Low CO₂** |
| WT | 31.6 | 53.9 | 30.9 | 182.0 |
| IL-2 | 26.6 | 39.2 | 32.2 | 61.1 |

The rate of CO₂ and of HCO₃⁻ uptake in *Synechococcus* sp. PCC 7942 and mutant IL-2 as affected by the concentration of CO₂ in the growth medium. The unidirectional CO₂ or HCO₃⁻ uptake of cells grown under high CO₂ conditions or exposed to low CO₂ for 12 hours is presented in µ mole Ci accumulated within the cells mg⁻¹ Chl h⁻¹. The results presented are the average of three different experiments, with four replicas in each experiment, the range of the data was within ±10 % of the average. WT - wild type.

Uptake of HCO₃- by wild type cells increased by approximately 6-fold following exposure to low CO₂ conditions for 12 hours. On the other hand, the same treatment resulted in only up to a 2-fold increase in HCO₃⁻ uptake for the IL-2 mutant. Uptake of CO₂ increased by approximately 50 % for both the wild type and the IL-2 mutant following transfer from high- to low CO₂ conditions. These data indicate that HCO₃⁻ transport and not CO₂ uptake was impaired in mutant IL-2.

The Vₘₐₓ of HCO₃⁻ uptake, estimated by MIMS [7, 22] at steady state photosynthesis (Figure 4a), were 220 and 290 µmol HCO₃⁻ mg⁻¹Chl h⁻¹ for high- and low-CO₂-grown wild type, respectively, and the corresponding K_{1/2} (HCO₃⁻) were 0.3 and 0.04 mM HCO₃⁻, respectively. These estimates are in close agreement with those reported earlier [7]. In high-CO₂-grown mutant IL-2, on the other hand, the HCO₃⁻ transporting system was apparently inactive. The curve relating the rate of HCO₃⁻ transport as a function of its concentration did not resemble the expected saturable kinetics (observed for the wild type), but was closer to a linear dependence as expected in a diffusion mediated process (Figure 4b). It was essential to raise the concentration of HCO₃⁻ in the medium to values as high as 25 mM in order to achieve rates of HCO₃⁻ uptake similar to the Vₘₐₓ depicted by the wild type.

The estimated Vₘₐₓ of CO₂ uptake by high-CO₂-grown wild type and IL-2 was similar for both at around 130-150 µmol CO₂ mg⁻¹ Chl h⁻¹ and the K_{1/2}(CO₂) values were around 5 µM (Figures 4a-b), indicating that CO₂ uptake was far less affected by the mutation in IL-2. Mutant cells that were exposed to low CO₂ for 12 hours showed saturable kinetics for HCO₃⁻ uptake suggesting the involvement of a carrier. However, the K_{1/2} (HCO₃⁻) was 4.5 mM HCO₃⁻ (i.e., 15- and 100-fold lower than in high- and in low-CO₂-grown wild type, respectively) and the Vₘₐₓ was approximately 200 µmol HCO₃- mg⁻¹ Chl h⁻¹. These data indicate the presence of a low affinity HCO₃⁻ transporter that is activated or utilized following inactivation of a high affinity HCO₃⁻ uptake in the mutant. The activity of the low affinity transporter resulted in the saturable transport kinetics observed in the low-CO₂-exposed mutant. These data further demonstrated that the mutant was able to respond to the low CO₂ signal.

The reason for the discrepancy between the data obtained by the two methods used, with respect to HCO₃⁻ uptake in wild type and mutant cells grown under high-CO₂-conditions, is not fully understood. It might be related to the fact that in the MIMS method HCO₃⁻ uptake is assessed as the difference between net photosynthesis and CO₂ uptake [6, 7, 22]. Therefore, at Ci concentrations below 3 mM, where the mutant did not exhibit net photosynthesis, HCO₃⁻ uptake was calculated as zero (Figures 4a-b). On the other hand, the filtering centrifugation technique, as used herein, measured the unidirectional HCO₃⁻ transport close to steady state via isotope exchange, which can explain some of the variations in the results. Not withstanding, the data obtained by both methods clearly indicates severe inhibition of HCO₃⁻ uptake in mutant cells exposed to low CO₂. It is interesting to note that while the characteristics of HCO₃⁻ uptake changed during acclimation of the mutant to low CO₂. CO₂ transport was not affected (Figures 4a-b). It is thus concluded that the high-CO₂-requiring phenotype of IL-2 is generated by the mutation of a HCO₃⁻ transporter rather than in non-acclimation to low CO₂.

### Genomic analysis of the IL-2 mutant:

Since IL-2 is impaired in HCO₃⁻ transport, it was used to identify and clone the relevant genomic region involved in the high affinity HCO₃⁻ uptake. Figure 1 presents a schematic map of the genomic region in *Synechococcus* sp. PCC 7942 where the insertion of the inactivating vector by a single cross over recombination event (indicated by a star) generated the IL-2 mutant. Sequence analysis (GenBank, accession No. U62616, SEQ ID NO:1) identified several open reading frames (identified in the legend of Figure 1), some are similar to those identified in *Synechocystis* PCC 6803 [23]. Comparison of the DNA sequence in the wild type with those in the two repeated regions (due to the single cross over) in mutant IL-2, identified several alterations in the latter. This included a deletion of 4 nucleotides in the *Apa*I side and a deletion of 6 nucleotides but the addition of one bp in the *Bam*HI side (Figure 5). The reason(s) for these alterations is not known, but they occurred during the single cross recombination between the genomic DNA and the supercoiled plasmid bearing the insert in the inactivation library. The high-CO₂-requiring phenotype of mutant JR12 of *Synechococcus* sp. PCC 7942 also resulted from deletions of part of the vector and of a genomic region, during a single cross over event, leading to a deficiency in purine biosynthesis under low CO₂ [24].

The alterations depicted in Figure 5 resulted in frame shifts which led to inactivation of both copies of ORF467 (nucleotides 2670-4073 of SEQ ID NO:1, SEQ ID NO:2) in IL-2. Insertion of a Kan^{r} cartridge within the EcoRV or *Nhe*I sites in ORF467, positions 2919 and 3897 (SEQ ID NO:1), respectively (indicated by the triangles in Figure 1), resulted in mutants capable of growing in the presence of kanamycin under low CO₂ conditions, though significantly (about 50 %) slower than the wild type. Southern analyses of these mutants clearly indicated that they were merodiploids, i.e., contained both the wild type and the mutated genomic regions.

Figures 2 and 3 show nucleic and amino acid alignments of ictB and slr1515, the most similar sequence to ictB identified in the gene bank, respectively. Note that the identical nucleotides shared between these nucleic acid sequences (Figure 2) equal 56 %, the identical amino acids shared between these amino acid sequences (Figure 3) equal 47 %, the similar amino acids shared between these amino acid sequences (Figure 3) equal 16 %, bringing the total homology therebetween to 63 % (Figure 3). When analyzed without the transmembrane domains, the identical amino acids shared between these amino acid sequences equal 40 %, the similar amino acids shared between these amino acid sequences equal 12 %, bringing the total homology therebetween to 52 %.

### EXAMPLE 2

### ictB - a putative inorganic carbon transporter

The protein encoded by ORF467 (SEQ ID NO:3) contains 10 putative transmembrane regions and is a membrane integrated protein. It is somewhat homologous to several oxidation-reduction proteins including the Na⁺/pantothenate symporter of *E. coli* (Accession No. P16256). Na⁺ ions are essential for HCO₃⁻ uptake in cyanobacteria and the possible involvement of a Na⁺/HCO₃⁻ symport has been discussed [3, 25, 26]. The sequence of the fourth transmembrane domain contains a region which is similar to the DCCD binding motif in subunit C of ATP synthase with the exception of the two outermost positions, replaced by conservative changes in ORF467. The large number of transport proteins that are homologous to the gene product of ORF467 also suggest that it is also a transport protein, possibly involved in HCO₃⁻ uptake. ORF467 is referred to herein as *ict*B (for inorganic carbon transport B [27]).

Sequence similarity between *cmpA,* encoding a 42-kDa polypeptide which accumulates in the cytoplasmic-membrane of low-CO₂-exposed *Synechococcus* PCC 7942 [28], and *nrtA* involved in nitrate transport [29], raised the possibility that CmpA may be the periplasmic part of an ABC-type transporter engaged in HCO₃⁻ transport [21, 42]. The role of the 42 kDa polypeptide, however, is not clear since inactivation of *cmpA* did not affect the ability of *Synechococcus* PCC7942 [30] and *Synechocystis* PCC6803 [21] to grow under a normal air level of CO₂ but growth was decreased under 20 ppm CO₂ in air [21]. It is possible that *Synechococcus* sp. PCC 7942 contains three different HCO₃⁻ carriers: the one encoded by *cmpA;* IctB; and the one expressed in mutant IL-2 cells exposed to low CO₂ whose identity is yet to be elucidated. These transporters enable the cell to maintain inorganic carbon supply under various environmental conditions.

### EXAMPLE 3

### Transgenic plants expressing ictB

The coding region of *ictB* was cloned downstream of a strong promoter (CaMV 35S) and downstream to, and in frame with, the transit peptide of pea rubisco small subunit. This expression cassette was ligated to vector sequences generating the construct shown in Figure 6.

*Arabidopsis thaliana* and Tobacco plants were transformed with the expression cassette described above using the Agrobacterium method. Seedlings of wild type and transgenic *Arabidopsis* plants were germinated and raised for 10 days under humid conditions. The seedlings were then transferred to pots, each containing one wild type and three transgenic plants. The pots were placed in two growth chambers (Binder, Germany) and grown at 20-21°C, 200 micromol photons m⁻² sec⁻¹ (9h:15h, light:dark). The relative humidity was maintained at 30-35% in one growth chamber and 70-75% in the other. In growth experiments, the plants were harvested from both growth chambers after 18 days of growth. The plants were quickly weighed (fresh weight) and dried in the oven overnight in order to determine the dry weight.

Northern analysis of plant RNA demonstrated that levels of *ictB* mRNA varied between different transgenic plants, while as expected, ictB mRNA was not detected in the Wild type plants (Figure 7).

Measurements of the photosynthetic characteristics with respect to CO₂ concentration showed that in both Tobacco (Figure 8) and Arabidopsis (not shown) the rate of photosynthesis at saturating CO₂ level was similar in the transgenic and wild type plants. On the other hand, under air levels of CO₂ or lower (such as experienced under water stress when the stomata are closed) the transgenic plants exhibited significantly higher photosynthetic rates than the wild type (Figure 8). Note that the slope of the curve relating photosynthesis to intercellular CO₂ concentration was steeper in the transgenic plants suggesting that the activity of Rubisco was higher in the transgenic plants.

### EXAMPLE 4

### Growth rate of ictB transgenic plants

In view of the positive effect of *ict*B expression on photosynthetic performance, the transgenic plants of the present invention were further tested for growth rates as compared to wild type plants (Figure 9).
Naturally, growth was faster in plants well supplied with water, maintained under the high (above 70%) relative humidity. Under such conditions there was no significant difference between the wild type and the transgenic plants.

On the other hand, the transgenic Arabidopsis plants grew significantly faster than the wild type under conditions of restricted water supply and low (lower than 40%) humidity (Figure 9). These data demonstrated the potential use of *ictB* to raise plant productivity particularly under dry conditions where stomatal closure may lead to lower intercellular CO₂ level and thus growth retardation.

The reasons for the very large effect of *ictB* expression on growth can be due to elevated CO₂ concentration at the site of Rubisco in the transgenic plants, consequent on enhanced HCO₃⁻ entry to the chloroplasts, would be expected to lower the compensation point for CO₂ and to lower the delta ¹³C of the organic matter produced [31]. Table 2 shows that the compensation point was slightly lower in the transgenic plants but the difference was not statistically significant. The slope of the curve relating photosynthesis to intercellular CO₂ concentration (Figure 8) was steeper in the transgenic plants suggesting (according to accepted models of photosynthesis [31-33]) that the activity of Rubisco was higher than in the wild type. Experiments where we compared the activity of Rubisco in transgenic and wild type plants suggested higher activity in the former (not shown).

**Table 2**

| ***The CO₂ compensation point and the fractionation of stable C isotopes in wild type and transgenic Arabidopsis and tobacco plants*** | | |
|---|---|---|
| **Sample** | **d¹³C** | **Compensation point** |
| *Arabidopsis* | | |
| A | 30.8 | 39 ± 4 |
| B | 31.6 | 41 ± 5 |
| WILD TYPE | 31.0 | 46 ± 5 |

| Tobacco | | |
|---|---|---|
| 3 | 30.8 | 47 ± 6 |
| 11 | 29.9 | 48 ± 7 |
| WILD TYPE | 27.35 | 57 ± 7 |

Thus, applying the teachings of the present invention one can transform plants such as C3 plants including, but not limited to, tomato, soybean, potato, cucumber, cotton, wheat, rice, barley and C4 crop plants, including, but not limited to, corn, sugar cane, sohrgum and others, to thereby generate plants which grow faster, and produce higher crop yield especially under limiting CO₂ and/or water limiting conditions.

### EXAMPLE 5

### ictB homologues

Two additional amino acid sequences exhibiting functional similarity to ictB are listed in Table 3 below. These sequences which encode polypeptides which are 75-80 % homologous to ictB (Table 4) can also be used to transform plants in order to achieve the resultant growth or yield enhancement described hereinabove.

**Table 3**

| Name | Putative/characterized function | Protein sequence SEQ ID NO: | Polynucleotide sequence SEQ ID NO: |
|---|---|---|---|
| *Anabaena* PCC7120 | none | 6 | 8 |
| *Nostoc punctiforme* | none | 7 | 9 |

**Table 4**

| ***Sequence comparison between ictBand hypothetical amino acid sequences from Synechocystis sp. PCC 6803, Anabaena PCC7120 and Nostoc punctiforme*** | | | | | |
|---|---|---|---|---|---|
| Organism | Putative/charac. function | Identical amino acids % | Similar amino acids % | Weakly similar amino acids % | Overall homology amino acids % |
| Synechocystis slr1515 | none | 46.41 | 19.41 | 10.13 | 75.95 |
| Anabaena | none | 51.37 | 18.32 | 9.68 | 79.37 |
| Nostoc | none | 50.84 | 18.28 | 11.55 | 80.67 |

### Expected commercial significance

On the basis of the results obtained with the transgenic Arabidopsis plants (see section 2, above), it is expected that expression of *ictB* in some of the most important crop plants including: wheat, rice, barley, potato, cotton, soybean, lettuce and tomato will lead to a significant increase in growth and commercial yield especially in regions in which commercial cultivation of food crops is substantially inhibited by growth conditions, such as for example the arid growth conditions characterizing various regions in Africa.

Although the invention has been described in conjunction with specific embodiments thereof, it is evident that many alternatives, modification and variations will be apparent to those skilled in the art. In addition, citation or identification of any reference in this application shall not be construed as an admission that such reference is available as prior art to the present invention.

### REFERENCES

1. Kaplan, A., Schwarz, R., Lieman-Hurwitz, J. and Reinhold, L. (1991) Plant Physiol. 97, 851-855.
2. Badger, M.R. and Price, D.G. (1994) Ann. Rev. Plant Physiol. Plant Mol. Biol. 45, 369-399.
3. Kaplan, A., Schwarz, R., Lieman-Hurwitz, J., Ronen-Tarazi, M. and Reinhold, L. (1994) in: The Molecular Biology of the Cyanobacteria (Bryant, D. Ed.), pp. 469-485, Kluwer Academic Pub., Dordrecht, The Netherlands.
4. Ronen-Tarazi, M., Schwarz, R., Bouevitch, A., Lieman-Hurwitz, J., Erez, J. and Kaplan, A. (1995) in: Molecular Ecology of Aquatic Microbes (Joint, I., Ed.), pp. 323-334. Springer-Verlag, Berlin.
5. Kaplan, A., Ronen-Tarazi, M., Zer, H., Schwarz, R., Tchernov, D., Bonfil, D.J., Schatz, D., Vardi, A., Hassidim, M. and Reinhold, L. (1998) Can. J.Bot. 76, 917-924.
6. Sultemeyer, D., Price, G.D., Bryant, D.A. and Badger, M.R. (1997) Planta 201, 36-42.
7. Sultemeyer, D., Klughammer, B., Badger, M.R. and Price, G.D. (1998) Plant Physiol. 116, 183-192.
8. Ronen-Tarazi, M., Shinder, V. and Kaplan, A. (1998) FEMS Microbiol Lett. 159, 317-324.
9. Sambrook, J., Fritsch, E.F. and Maniatis, T. (1989) Molecular Cloning. A Laboratory Manual. Cold Spring Harbor Laboratory Press, Cold Spring Harbor, New York.
10. Kaplan, A., Marcus, Y. and Reinhold, L. (1988) in: Methods in Enzymology (Packer, L. and Glazer, A.N., Eds.), pp. 534-539. Academic Press, New York.
11. Tchernov, D., Hassidim, M., Luz, B., Sukenik, A., Reinhold, L. and Kaplan, A. (1997) Curr. Biol. 7, 723-728.
12. Badger, M.R. and Price, G.D. (1992) Physiol. Plant. 84,606-615.
13. Ronen-Tarazi, M., Lieman-Hurwitz, J., Gabay, C., Orus, M. and Kaplan, A. (1995) Plant Physiol. 108, 1461-1469.
14. Dolganov, N. and Grossman, A. (1993) J. Bacteriol. 175,7644-7651.
15. Ronen-Tarazi, M., Bonfil, D.J., Schatz, D. and Kaplan, A. (1998) Can. J. Bot. 76, 942-948.
16. Marcus, Y., Schwarz, R., Friedberg, D. and Kaplan, A. (1986) Plant Physiol. 82, 610-612.
17. Schwarz, R., Friedberg, D., Reinhold, L. and Kaplan, A. (1988) Plant Physiol. 88, 284-288.
18. Fukuzawa, H., Suzuki, E., Komukai, Y. and Miyachi, S. (1992) Proc. Natl. Acad. Sci. USA 89,4437-4441.
19. Schwarz, R., Reinhold, L. and Kaplan, A. (1995) Plant Physiol. 108, 183-190.
20. Marco, M., Ohad, N., Schwarz, R., Lieman-Hurwitz, J., Gabay, C. and Kaplan, A. (1993) Plant Physiol. 101, 1047-1053.
21. Ohkawa, H., Sonoda, M., Katoh, H. and Ogawa, T. (1998) in: Proceedings of The Third International Symposium on Inorganic Carbon Uptake by Aquatic Photosynthetic Organisms (Colman, B. Ed.), Can J Bot 76, 1035-1042.
22. Yu, J.W., Price, G.D. and Badger, M.R. (1994) Aust. J. Plant Physiol. 21, 185-195.
23. Kaneko, T., Sato, S., Kotani, H., Tanaka, A., Asamizu, E., Nakamura, Y., Miyajima, N., Hirosawa, M., Sugiura, M., Sasamoto, S., Kimura, T., Hosouchi, T., Matsuno, A., Muraki, A., Nakazaki, N., Naruo, K., Okumura, S., Shimpo, S., Takeuchi, C., Wada, T., Watanabe, A., Yamada, M., Yasuda, M. and Tabata, S. (1996) DNA Res. 3, 109-136.
24. Lieman-Hurwitz J., Schwarz, R., Martinez, F., Maor, Z., Reinhold, L., and Kaplan, A. (1990) in: Proceedings of The Second International Symposium on Inorganic Carbon Uptake by Aquatic Photosynthetic Organisms (Colman, B. Ed.), Can J Bot 69: 945-950.
25. Kaplan, A., Schwarz, R., Ariel, R., and Reinhold, L. (1990) in: Regulation of Photosynthetic Processes (Kanay, R., Katoh, R.S. and Miyachi, S. Eds.), Special Issue of the Botanical Magazine, vol. 2, pp. 53-71, Tokyo.
26. Tyrrell, P. N., Kandasamy, R. A., Crotty, C. M. and Espie, G. S. (1996) Plant Physiol. 112, 79-88
27. Ogawa, T. (1991) Plant Physiol. 96, 280-284.
28. Omata, T. and Ogawa, T. (1986) Plant Physiol. 80, 525-530.
29. Omata, T. (1992) in: Research in Photosynthesis (Murata, N. Ed.) vol. III, 807-810, Kluwer Academic Pub., Dodrecht, The Netherlands.
30. Omata, T., Carlson, T.J., Ogawa, T. and Pierce, J. (1990) Plant Physiol. 93, 305-311.
31. Woodrow, I.E. and Berry, J.A. (1988) Ann. Rev. Plant Physiol 39, 533-594.
32. Mott, K.A. and Woodrow, I.E. (2000) Journal of Experimental Botany 51, 399-406.
33. Poolman, M.G., Fell, D.A. and Thomas, S. (2000) Journal of Experimental Botany 51, 319-328.
34. Kaplan, A. and Reinhold, L. (1999) Annu. Rev. Plant Physiol. Plant Mol. Biol. 50, 539-570.
35. Spreitzer, R.J. (1999) Photosynthesis Research 60, 29-42.
36. Horton, P. (2000) Journal of Experimental Botany 51, 475-485.
37. Matsuoka, M., Nomura, M., Agarie, S., Miyao-Tokutomi, M. and Ku, M.S.B. (1998) Journal of Plant Research 111, 333-337.
38. Nomura, M. et al. (2000) Plant Mol. Biol. 44, 99-106.
39. Takeuchi, K., Akagi, H., Kamasawa, N., Osumi, M. and Honda, H. (2000) Planta 211, 265-274.
40. Suzuki, S., Murai, N., Burnell, J.N. and Arai, M. (2000) Plant Physiol. 124,163-172.
41. Bonfil, D.J., Ronen-Tarazi, M., Sultemeyer, D.; Lieman-Hurwitz, J., Schatz, D. and Kaplan, A. (1998) FEBS Lett. 430, 236-240.
42. Maeda, S-I., Price, G. D., Badger, M. R., Enomoto, C. and Omata, T. (2000) J. Biol. Chem. 275, 20551-20555

## Claims

1. A method of enhancing growth and/or commercial yield of a plant, the method comprising expressing within the plant a polypeptide including an amino acid sequence at least 60% homologous to that set forth in SEQ ID NOs: 5, 6 or 7.

2. A method of enhancing growth and/or commercial yield of a plant, the method comprising expressing within the plant a polypeptide including an amino acid sequence at least 60% homologous to that set forth in SEQ ID NOs: 3, 5, 6 or 7, wherein the plant is grown in an environment **characterized by** humidity lower than 40%.

3. The method of claim 1 or 2, wherein the plant is grown in an environment **characterized by** an intercellular CO₂ concentration lower then 10 micromolar.

4. The method of anyone of claims 1 to 3, wherein expressing said polypeptide within the plant is effected by transforming at least a portion of the plant cells with a nucleic acid construct including a polynucleotide region encoding said polypeptide

5. The method of claim 4, wherein said transforming is effected by a method selected from the group consisting of agrobacterium mediated transformation, viral infection, electroporation and particle bombardment.

6. The method of anyone of claims 2 to 5, wherein said amino acid sequence is as set forth by SEQ ID NOs: 3, 5, 6 or 7.

7. The method of claim 4, wherein said nucleic acid construct further includes a second polynucleotide region encoding a transit peptide.

8. The method of claim 4, wherein said nucleic acid construct further includes a promoter sequence for directing transcription of said first polynucleotide region.

9. The method of claim 4, wherein said nucleic acid construct further includes a promoter sequence for directing transcription of said first and said second polynucleotide regions.

10. The method of claim 8, wherein said promoter is functional in eukaryotic cells.

11. The method of claim 10, wherein said promoter is selected from the group consisting of a constitutive promoter, an inducible promoter, a developmentally regulated promoter and a tissue specific promoter.

12. The method of anyone of claims 1 to 11, wherein said plant is a C3 plant.

13. The method of claim 12, wherein said C3 plant is selected from the group consisting of tomato, soybean, potato, cucumber, cotton, wheat, rice, barley, lettuce, solidago, banana and poplar.

14. The method of anyone of claims 1 to 11, wherein said plant is a C4 plant.

15. The method of claim 14, wherein said C4 plant is selected from the group consisting of corn, sugar cane, and sorghum.

16. The method of anyone of claims 1 to 15, wherein the plant expressing said polypeptide is **characterized by** a growth rate which is at least 10% higher than that of a similar plant not expressing said polypeptide when both are grown under similar growth conditions.

17. The method of claim 16, wherein said growth rate is determined by at least one growth parameter selected from the group consisting of increased fresh weight, increased dry weight, increased root growth, increased shoot growth and increased flower development over time.

18. A transformed plant expressing a polypeptide including an amino acid sequence at least 60% homologous to that set forth in SEQ ID NOs: 5, 6 or 7, said transformed plant **characterized by** an enhanced growth as compared to similar non transformed plant grown under similar growth conditions.

19. The transformed plant of claim 18, wherein said growth conditions include humidity conditions of less than 40%.

20. The transformed plant of claim 18 or 19, wherein said amino acid sequence is as set forth by SEQ ID NOs: 5, 6 or 7.

21. The transformed plant of anyone of claims 18 to 20, wherein said transformed plant is a C3 plant.

22. The transformed plant of claim 21, wherein said C3 plant is selected from the group consisting of tomato, soybean, potato, cucumber, cotton, wheat, rice, barley, lettuce, solidago, banana, poplar, and citrus.

23. The transformed plant of anyone of claim 18 to 20, wherein said transformed plant is a C4 plant.

24. The transformed plant of claim 23, wherein said C4 plant is selected from the group consisting of corn, sugar cane, and sorghum.

25. The transformed plant of anyone of claim 18 to 24, wherein a growth rate of said transformed plant is at least 10% higher than that of a similar non transformed plant when both are grown under similar growth conditions.

26. The transformed plant of claim 25, wherein said growth rate is determined by at least one growth parameter selected from the group consisting of fresh weight, dry weight, root growth, shoot growth and flower development.

27. The transformed plant of anyone of claims 18 to 25, wherein said transformed plant is further **characterized by** an increased commercial yield as compared to similar non transformed plant grown under similar conditions.

28. The transformed plant of anyone of claims 18 to 25, wherein said growth conditions include water stress, low humidity, salt stress, and/or low CO₂ conditions.

29. A nucleic acid expression construct comprising:
(a) a first polynucleotide region encoding a polypeptide including an amino acid sequence at least 60% homologous to that set forth by SEQ ID NOs: 5, 6 or 7; and
(b) a second polynucleotide region functional as a promoter and being for directing the transcription of said first polynucleotide region in eukaryotic cells.

30. The nucleic acid expression construct of claim 29, wherein said promoter is selected from the group consisting of a constitutive promoter, an inducible promoter, a developementally regulated promoter and a tissue specific promoter.

31. The nucleic acid expression construct of claim 29, wherein said promoter is a plant promoter.

32. The nucleic acid expression construct of claim 29, wherein said first polynucleotide region further encodes a transit peptide being translationally fused to said polypeptide.

## Patentansprüche

1. Verfahren zum Steigern des Wachstums und/oder des wirtschaftlichen Ertrags einer Pflanze, umfassend Exprimieren eines Polypeptids, das eine Aminosäuresequenz enthält, die mindestens 60% homolog zu derjenigen in SEQ ID NR: 5, 6 oder 7 ist, in der Pflanze.

2. Verfahren zum Steigern des Wachstums und/oder des wirtschaftlichen Ertrags einer Pflanze, umfassend Exprimieren eines Polypeptids, das eine Aminosäuresequenz enthält, die mindestens 60% homolog zu derjenigen in SEQ ID NR: 3, 5, 6 oder 7 ist, in der Pflanze, wobei die Pflanze in einer Umgebung gezogen wird, die durch eine Luftfeuchtigkeit von weniger als 40% **gekennzeichnet** ist.

3. Verfahren nach Anspruch 1 oder 2, wobei die Pflanze in einer Umgebung gezogen wird, die durch eine interzelluläre CO₂-Konzentration von weniger als 10 mikromolar **gekennzeichnet** ist.

4. Verfahren nach einem der Ansprüche 1 bis 3, wobei das Exprimieren des Polypeptids in der Pflanze durch Transformieren mindestens eines Anteils der Pflanzenzellen mit einem Nukleinsäurekonstrukt durchgeführt wird, das eine das Polypeptid kodierende Polynukleotidregion enthält.

5. Verfahren nach Anspruch 4, wobei das Transformieren mit einem Verfahren ausgewählt aus der Gruppe bestehend aus Agrobacterium-vermittelter Transformation, viraler Infektion, Elektroporation und Beschuss mit Partikeln durchgeführt wird.

6. Verfahren nach einem der Ansprüche 2 bis 5, wobei die Aminosäuresequenz die der SEQ ID NR: 3, 5, 6 oder 7 ist.

7. Verfahren nach Anspruch 4, wobei das Nukleinsäurekonstrukt ferner eine zweite Polynukleotidregion enthält, die ein Transitpeptid kodiert.

8. Verfahren nach Anspruch 4, wobei das Nukleinsäurekonstrukt ferner eine Promotersequenz zum Steuern der Transkription der ersten Polynukleotidregion enthält.

9. Verfahren nach Anspruch 4, wobei das Nukleinsäurekonstrukt ferner eine Promotersequenz zum Steuern der Transkription der ersten und zweiten Polynukleotidregion enthält.

10. Verfahren nach Anspruch 8, wobei der Promoter in eukaryotischen Zellen funktionell ist.

11. Verfahren nach Anspruch 10, wobei der Promoter ausgewählt ist aus der Gruppe bestehend aus einem konstitutiven Promoter, einem induzierbaren Promoter, einem entwicklungsregulierten Promoter und einem gewebespezifischen Promoter.

12. Verfahren nach einem der Ansprüche 1 bis 11, wobei die Pflanze eine C3-Pflanze ist.

13. Verfahren nach Anspruch 12, wobei die C3-Pflanze ausgewählt ist aus der Gruppe bestehend aus Tomate, Sojabohne, Kartoffel, Gurke, Baumwolle, Weizen, Reis, Gerste, Salat, Goldrute, Banane und Pappel.

14. Verfahren nach einem der Ansprüche 1 bis 11, wobei die Pflanze eine C4-Pflanze ist.

15. Verfahren nach Anspruch 14, wobei die C4-Pflanze ausgewählt ist aus der Gruppe bestehend aus Mais, Zuckerrohr und Hirse.

16. Verfahren nach einem der Ansprüche 1 bis 15, wobei die das Polypeptid exprimierende Pflanze durch eine Wachstumsrate **gekennzeichnet** ist, die mindestens 10% höher ist als diejenige einer gleichen Pflanze, die das Polypeptid nicht exprimiert, wenn beide unter gleichen Wachstumsbedingungen gezogen werden.

17. Verfahren nach Anspruch 16, wobei die Wachstumsrate durch mindestens einen Wachstumsparameter ausgewählt aus der Gruppe bestehend aus erhöhtem Frischgewicht, erhöhtem Trockengewicht, erhöhtem Wurzelwachstum, erhöhtem Sprossenwachstum und gesteigerter Blütenentwicklung über die Zeit bestimmt wird.

18. Transformierte Pflanze, die ein Polypeptid exprimiert, das eine Aminosäuresequenz enthält, die mindestens 60% homolog zu derjenigen in SEQ ID NR: 5, 6 oder 7 ist, wobei die transformierte Pflanze durch ein erhöhtes Wachstum im Vergleich zu einer gleichen, nicht-transformierten Pflanze, die unter gleichen Wachstumsbedingungen gezogen wird, **gekennzeichnet** ist.

19. Transformierte Pflanze nach Anspruch 18, wobei die Wachstumsbedingungen Feuchtigkeitsbedingungen von weniger als 40% beinhalten.

20. Transformierte Pflanze nach Anspruch 18 oder 19, wobei die Aminosäuresequenz diejenige der SEQ ID NR: 5, 6 oder 7 ist.

21. Transformierte Pflanze nach einem der Ansprüche 18 bis 20, wobei die transformierte Pflanze eine C3-Pflanze ist.

22. Transformierte Pflanze nach Anspruch 21, wobei die C3-Pflanze ausgewählt ist aus der Gruppe bestehend aus Tomate, Sojabohne, Kartoffel, Gurke, Baumwolle, Weizen, Reis, Gerste, Salat, Goldrute, Banane, Pappel und Zitrone.

23. Transformierte Pflanze nach einem der Ansprüche 18 bis 20, wobei die transformierte Pflanze eine C4-Pflanze ist.

24. Transformierte Pflanze nach Anspruch 23, wobei die C4-Pflanze ausgewählt ist aus der Gruppe bestehend aus Mais, Zuckerrohr und Hirse.

25. Transformierte Pflanze nach einem der Ansprüche 18 bis 24, wobei die Wachstumsrate der transformierten Pflanze mindestens 10% höher ist als diejenige einer gleichen, nicht-transformierten Pflanze, wenn beide unter gleichen Wachstumsbedingungen gezogen werden.

26. Transformierte Pflanze nach Anspruch 25, wobei die Wachstumsrate durch mindestens einen Wachstumsparameter ausgewählt aus der Gruppe bestehend aus Frischgewicht, Trockengewicht, Wurzelwachstum, Sprossenwachstum und Blütenentwicklung bestimmt wird.

27. Transformierte Pflanze nach einem der Ansprüche 18 bis 25, wobei die transformierte Pflanze weiter **gekennzeichnet ist durch** einen erhöhten wirtschaftlichen Ertrag im Vergleich zu einer gleichen, nicht-transformierten Pflanze, die unter gleichen Bedingungen gezogen wird.

28. Transformierte Pflanze nach einem der Ansprüche 18 bis 25, wobei die Wachstumsbedingungen Wasserstress, niedrige Feuchtigkeit, Salzstress und/oder niedrige CO₂-Bedingungen einschließen.

29. Nukleinsäureexpressionskonstrukt umfassend:
(a) eine erste Polynukleotidregion, die ein Polypeptid kodiert, das eine Aminosäuresequenz enthält, die mindestens 60% homolog zu derjenigen der SEQ ID NR: 5, 6 oder 7 ist; und
(b) eine zweite Polynukleotidregion, die als Promoter funktioniert und zur Steuerung der Transkription der ersten Polynukleotidregion in eukaryotischen Zellen vorgesehen ist.

30. Nukleinsäureexpressionskonstrukt nach Anspruch 29, wobei der Promoter ausgewählt ist aus der Gruppe bestehend aus einem konstitutiven Promoter, einem induzierbaren Promoter, einem entwicklungsregulierten Promoter und einem gewebespezifischen Promoter.

31. Nukleinsäureexpressionskonstrukt nach Anspruch 29, wobei der Promoter ein Pflanzenpromoter ist.

32. Nukleinsäureexpressionskonstrukt nach Anspruch 29, wobei die erste Polynukleotidregion ferner ein Transitpeptid kodiert, das translational an das Polypeptid fusioniert ist.

## Revendications

1. Procédé d'amplification de la croissance et/ou du rendement commercial d'une plante, le procédé comprenant l'expression au sein de la plante d'un polypeptide comprenant une séquence d'acides aminés homologue à au moins 60 % avec celle représentée par SEQ ID NO : 5, 6 ou 7.

2. Procédé d'amplification de la croissance et/ou du rendement commercial d'une plante, le procédé comprenant l'expression au sein de la plante d'un polypeptide comprenant une séquence d'acides aminés homologue à au moins 60 % avec celle représentée par SEQ ID NO : 3, 5, 6 ou 7, dans lequel la plante est cultivée dans un environnement **caractérisé par** une humidité inférieure à 40 %.

3. Procédé selon la revendication 1 ou 2, dans lequel la plante est cultivée dans un environnement **caractérisé par** une concentration de CO₂ intercellulaire inférieure à 10 micromoles.

4. Procédé selon l'une quelconque des revendications 1 à 3, dans lequel l'expression dudit polypeptide au sein de la plante est effectuée par la transformation d'au moins une partie des cellules végétales avec un construit d'acide nucléique comprenant une région polynucléotidique codant pour ledit polypeptide.

5. Procédé selon la revendication 4, dans lequel ladite transformation est effectuée par un procédé choisi dans le groupe constitué d'une transformation médiée par une agrobactérie, une infection virale, une électroporation et un bombardement de particules.

6. Procédé selon l'une quelconque des revendications 2 à 5, dans lequel ladite séquence d'acides aminés est telle que représentée par SEQ ID NO : 3, 5, 6 ou 7.

7. Procédé selon la revendication 4, dans lequel ledit construit d'acide nucléique comprend en outre une seconde région polynucléotidique codant pour un peptide de transit.

8. Procédé selon la revendication 4, dans lequel ledit construit d'acide nucléique comprend en outre une séquence promotrice pour diriger la transcription de ladite première région polynucléotidique.

9. Procédé selon la revendication 4, dans lequel ledit construit d'acide nucléique comprend en outre une séquence promotrice pour diriger la transcription desdites première et seconde régions polynucléotidiques.

10. Procédé selon la revendication 8, dans lequel ledit promoteur est fonctionnel dans des cellules eucaryotes.

11. Procédé selon la revendication 10, dans lequel ledit promoteur est choisi dans le groupe constitué d'un promoteur constitutif, d'un promoteur inductible, d'un promoteur régulé au cours du développement et d'un promoteur à spécificité tissulaire.

12. Procédé selon l'une quelconque des revendications 1 à 11, dans lequel ladite plante est une plante en C3.

13. Procédé selon la revendication 12, dans lequel ladite plante en C3 est choisie dans le groupe constitué de tomate, soja, pomme de terre, concombre, coton, blé, riz, orge, laitue, solidage, banane et peuplier.

14. Procédé selon l'une quelconque des revendications 1 à 11, dans lequel ladite plante est une plante en C4.

15. Procédé selon la revendication 14, dans lequel ladite plante en C4 est choisie dans le groupe constitué de maïs, canne à sucre et sorgho.

16. Procédé selon l'une quelconque des revendications 1 à 15, dans lequel la plante exprimant ledit polypeptide est **caractérisée par** une vitesse de croissance qui est au moins 10 % supérieure à celle d'une plante similaire n'exprimant pas ledit polypeptide lorsque toutes les deux sont cultivées dans des conditions de croissance similaires.

17. Procédé selon la revendication 16, dans lequel ladite vitesse de croissance est déterminée par au moins un paramètre de croissance choisi dans le groupe constitué de l'augmentation du poids frais, l'augmentation du poids sec, l'augmentation de la croissance des racines, l'augmentation de la croissance des pousses et l'augmentation du développement des fleurs dans le temps.

18. Plante transformée exprimant un polypeptide comprenant une séquence d'acides aminés homologue à au moins 60 % avec celle représentée par SEQ ID NO : 5, 6 ou 7, ladite plante transformée **caractérisée par** une amplification de la croissance par comparaison à une plante non transformée similaire cultivée dans des conditions de croissance similaires.

19. Plante transformée selon la revendication 18, où lesdites conditions de croissance comprennent des conditions d'humidité inférieures à 40 %.

20. Plante transformée selon la revendication 18 ou 19, dans laquelle ladite séquence d'acides aminés est telle que représentée par SEQ ID NO : 5, 6 ou 7.

21. Plante transformée selon l'une quelconque des revendications 18 à 20, où ladite plante transformée est une plante en C3.

22. Plante transformée selon la revendication 21, où ladite plante en C3 est choisie dans le groupe constitué de tomate, soja, pomme de terre, concombre, coton, blé, riz, orge, laitue, solidage, banane, peuplier et agrume.

23. Plante transformée selon l'une quelconque des revendications 18 à 20, où ladite plante transformée est une plante en C4.

24. Plante transformée selon la revendication 23, où ladite plante en C4 est choisie dans le groupe constitué de maïs, canne à sucre et sorgho.

25. Plante transformée selon l'une quelconque des revendications 18 à 24, où la vitesse de croissance de ladite plante transformée est au moins 10 % supérieure à celle d'une plante non transformée similaire lorsque toutes les deux sont cultivées dans des conditions de croissance similaires.

26. Plante transformée selon la revendication 25, où ladite vitesse de croissance est déterminée par au moins un paramètre de croissance choisi dans le groupe constitué du poids frais, du poids sec, de la croissance des racines, de la croissance des pousses et du développement des fleurs.

27. Plante transformée selon l'une quelconque des revendications 18 à 25, où ladite plante transformée est en outre **caractérisée par** une augmentation du rendement commercial par comparaison à une plante non transformée similaire cultivée dans des conditions similaires.

28. Plante transformée selon l'une quelconque des revendications 18 à 25, où lesdites conditions de croissance comprennent des conditions de stress hydrique, de faible humidité, de stress salin et/ou de CO₂ faibles.

29. Construit d'expression d'acide nucléique comprenant
(a) une première région polynucléotidique codant pour un polypeptide comprenant une séquence d'acides aminés homologue à au moins 60 % avec celle représentée par SEQ ID NO : 5, 6 ou 7 ; et
(b) une seconde région polynucléotidique fonctionnelle en tant que promoteur et servant à diriger la transcription de ladite première région polynucléotidique dans des cellules eucaryotes.

30. Construit d'expression d'acide nucléique selon la revendication 29, dans lequel ledit promoteur est choisi dans le groupe constitué d'un promoteur constitutif, d'un promoteur inductible, d'un promoteur régulé au cours du développement et d'un promoteur à spécificité tissulaire.

31. Construit d'expression d'acide nucléique selon la revendication 29, dans lequel ledit promoteur est un promoteur végétal.

32. Construit d'expression d'acide nucléique selon la revendication 29, dans lequel ladite première région polynucléotidique code en outre pour un peptide de transit qui est fusionné par traduction au dit polypeptide.
